# EUROPEAN PATENT APPLICATION

(11) **EP 2 617 434 A1**
(43) Date of publication of application: **24.07.2013**
(21) Application number: 12382016.9
(22) Date of filing: 20.01.2012
(51) Int. Cl.: A61K 39/12

(54) **HIV-1 integrase deficient immunogens and methods for loading dendritic cells with said immunogens**

(71) Applicant: Laboratorios del. Dr. Esteve, S.A., 08041 Barcelona (ES); Fundació Privada Institut de Recerca de la SIDA-Caixa, 08916 Badalona (ES); Institució Catalana de Recerca i Estudis Avançats, 08010 Barcelona (ES)
(72) Inventor: Buzón, José, 02140 Cambridge, Massachusetts (US); Martínez-Picado, Javier, 08340 Vilassar de Mar Barcelona (ES)
(74) Representative: ABG Patentes, S.L.

(57) **Abstract**

The present invention relates to a method for obtaining an antigen-loaded dendritic cell *in vitro* comprising contacting an immature dendritic cell with an immunogen, preferably a HIV immunogen, and simultaneously, during at least part of said step, placing said immature dendritic cell under conditions adequate for maturation. The invention relates also to HIV-1 immunogens lacking a functional integrase protein.

## Description

### FIELD OF THE INVENTION

The present invention relates to methods for obtaining an antigen-loaded antigen-presenting cell, particularly a dendritic cell loaded with a HIV-1 immunogen. The invention also relates to HIV-1 immunogens lacking a functional integrase protein and uses thereof.

### BACKGROUND OF THE INVENTION

Dendritic cells (DCs) are the most potent antigen presenting cells in the immune system, which link innate and adaptive immunity. They capture pathogens in the mucosa and then migrate to the secondary lymphoid tissue, where they acquire the mature phenotype required to induce efficiently adaptive immune responses. After reaching the T cell areas of the lymph nodes, mature DCs can present pathogen-derived peptides in association with human leukocyte antigen (HLA) system molecules to naive T cells. The HLA system is the major histocompatibility complex (MHC) in human beings. This process initiates a cellular immune response that involves CD4+ T helper cells, CD8+ cytotoxic T lymphocytes (CTL) and humoral immune response with the activation of B cells. Nevertheless, DCs have also been proposed to contribute to HIV-1 spread during HIV-1 exposure *in vivo.* DCs are among the first target cells to encounter and be infected by HIV-1, as they are abundant at the most common sites for viral entry such as the genital and anal mucosae. From here they move to lymphoid tissues where they interact and infect CD4+ T cells, the main target of HIV-1. *See* Granelli-Piperno A, et al., J. Virol. 1998; 72:2733-2737.

Several works have demonstrated that upon maturation, DCs improve their efficiency to transmit HIV-1 through trans-infection, a process where DCs retain and transfer infectious virions without becoming infected themselves. Generally, all maturation signals upregulate phenotypic markers for HLA and co-stimulatory molecules, but functional ability of the resulting mature DCs varies. Consequently, depending on their qualitative maturation state, DCs are able to polarize various T cell responses.

The ability of mature DCs to transfer HIV is also largely influenced by the maturation stimuli and the resulting DC subsets. However, previous studies have observed that DCs matured with lipopolysaccharide (LPS), both monocyte-derived DCs (mDCs) and blood-derived myeloid DCs, capture higher amounts of HIV-1 compared to immature monocyte-derived DCs (iDCs) or immature myeloid DCs. This has also been found to be correlated with a greater ability to transfer HIV-1 to susceptible target cells.

LPS maturation of DCs also upregulates the capture of exosomes, secreted cellular vesicles of endocytic origin with an important role in intercellular communication. Exosomes and HIV-1 are biochemically connected, with similarities in size, composition, biogenesis and cellular release. In addition, it has been described that exosomes and HIV-1 use an identical entry pathway into LPS-matured DCs, colocalizing in the same CD81+ intracellular compartment. Thus, several lines of evidence suggest that HIV-1 particles may exploit the exosome-antigen dissemination pathway intrinsic to mature DC for mediating trans-infection of T lymphocytes. Exosomes have also been shown to contribute to antigen exchange and T cell activation.

DCs may play a dual role in HIV-1 infection: increasing HIV-1 dissemination while triggering an adaptive response against viral infection. Although it is well documented that the higher viral capture by LPS-matured DCs results in an increased trans-infection to target cells, little is known about the antigen presentation abilities of this DC subset.

Notwithstanding the above, there exists a long-felt and continuing need in the art for HIV immunogens and more effective methods for loading dendritic cells with immunogens, particularly with HIV immunogens, that may support better the preparation of safer and more efficient autologous HIV therapeutic vaccines.

### SUMMARY OF THE INVENTION

In a first aspect, the invention relates to a method, hereinafter referred to as the "first method of the invention", for obtaining an antigen-loaded antigen-presenting cell *in vitro* comprising contacting an immature dendritic cell with an immunogen comprising said antigen under conditions adequate for processing of the immunogen and presentation by the antigen-presenting cell.

In a second aspect, the invention relates to an antigen-loaded antigen-presenting cell obtainable by the method according to the first aspect of the invention.

In further aspects, the invention relates to an antigen-presenting cell according to the invention for use in medicine as well as for use in the treatment or prevention of a disease which requires an immune response against the antigen which is loaded in the antigen-presenting cell.

In another aspect, the invention relates to an immunogenic composition or a vaccine comprising an antigen-presenting cell according to the invention.

In another aspect, the invention relates to a HIV-1 viral particle lacking a functional integrase protein that contains a deletion in the integrase protein or lacks the integrase protein.

In further aspects, the invention relates to a HIV-1 viral genome characterized by lacking partially or completely the integrase coding region, a vector comprising the HIV-1 viral genome characterized by lacking partially or completely the integrase coding region or a cell comprising a HIV-1 viral genome characterized by lacking partially or completely the integrase encoding region or a vector comprising a HIV-1 viral genome characterized by lacking partially or completely the integrase encoding region for use in medicine as well as for use in the treatment or prevention of a HIV infection or a disease associated with a HIV infection.

In another aspect, the invention relates to a method (hereinafter referred to as the "second method of the invention") for obtaining a dendritic cell loaded with a HIV-1 immunogen *in vitro* comprising loading a dendritic cell with a HIV-1 viral particle lacking a functional integrase protein.

In another aspect, the invention relates to a dendritic cell loaded with a HIV-1 viral particle lacking a functional integrase protein obtainable by the second method of the invention.

In another aspect, the invention relates to a dendritic cell according to the invention for use in medicine as well as for use in the treatment or prevention of a HIV infection or a disease associated with a HIV infection.

In another aspect, the invention relates to an immunogenic composition or a vaccine comprising a dendritic cell according to the invention.

### DEPOSIT OF MICROORGANISMS

The plasmid pNL4-3ΔIN (pNL4-3-Delta-Integrase) was deposited on July 22^{nd}, 2010, under accession number DSM 23817 at the DSMZ (Deutsche Sammlung von Mikroorganismen und Zellkulturen GmbH), Inhoffenstraße 7 B, D-38124 Braunschweig, Federal Republic of Germany.

### DESCRIPTION OF THE FIGURES

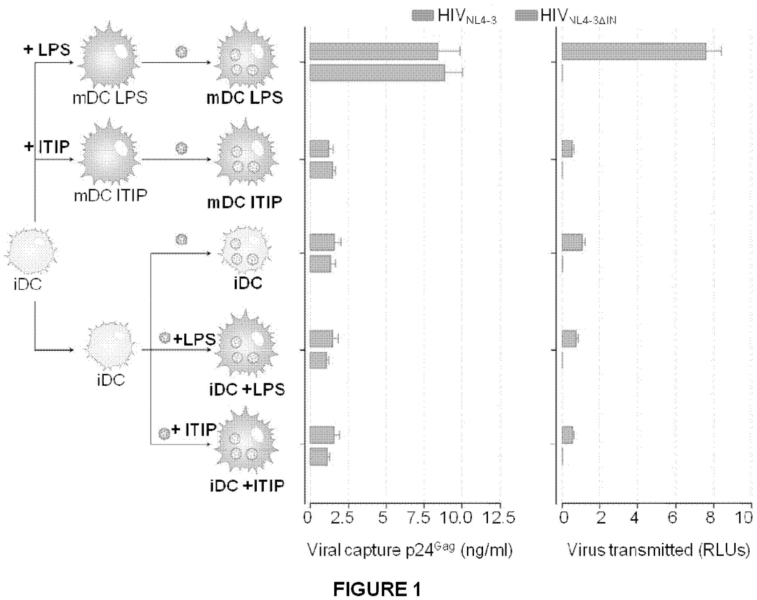

**Figure 1****.** Viral capture and viral transmission of HIV_{NL4-3} and HIV_{NL4-3ΔIN} in different cell conditions. Left panel: Protocol for HIV-1 capture assay of fully mature dendritic cells (DCs) that were matured with LPS (mDC LPS) or with ITIP (mDC ITIP) for 48 h prior to viral incubation; and for immature DCs (iDCs) and iDCs that were matured with LPS (iDC+LPS) or with ITIP (iDC+ITIP) during viral incubation. Middle panel: Comparative capture of HIV_{NL4-3} and HIV_{NL4-3ΔIN} by each cell condition described in left panel. Determination of DC-associated HIV-1 by p24^{Gag} ELISA was performed after a 6 h viral incubation at 37°C. Right panel: Transmission of HIV_{NL4-3} and HIV_{NL4-3ΔIN} captured by each cell condition to reporter cell line TZM-bl. Data show mean values and standard error of the mean (SEM) from three independent experiments including cells from at least seven different donors. LPS: lipopolysaccharide; ITIP: IL-1β, TNF-α, IL-6 and PGE₂; RLUs: relative light units.

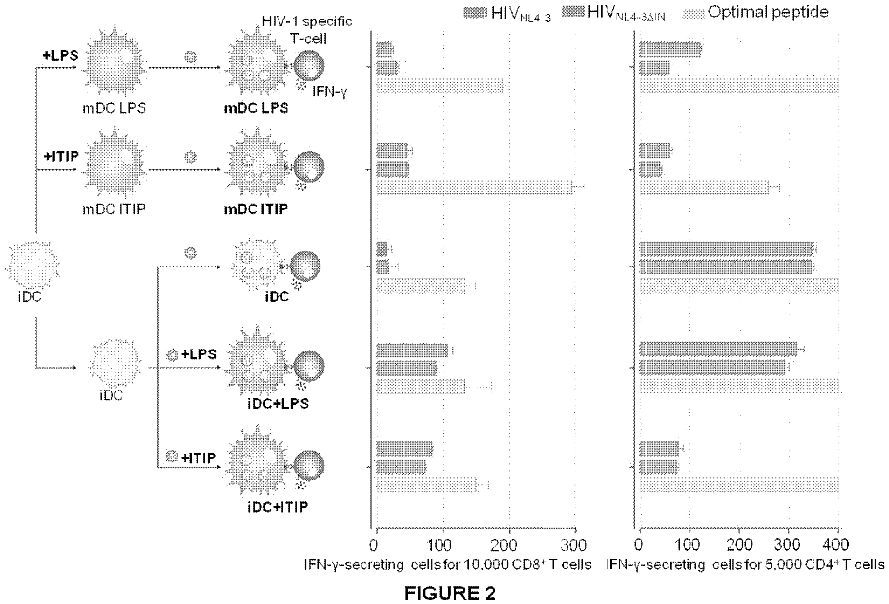

**Figure 2****.** Comparative analysis of the interferon gamma (IFN-γ) production by the EM40-F21 HIV p17^{Gag} SL9 CD8⁺ T cell clone and the N2 HIV p24^{Gag} CD4⁺ T cell clone in antigen presentation assays in each dendritic cell (DC) condition tested in Figure 1. Left panel: Protocol for HLA-I and HLA-II antigen presentation assays of immature DCs (iDCs), iDCs cultured with ITIP (iDC+ITIP) or with LPS (iDC+LPS) during viral incubation, and fully mature DCs cultured with ITIP (mDC ITIP) or with LPS (mDC LPS) for 48 h prior to viral incubation. Middle panel: Comparative HIV-specific CD8⁺ activation by each cell condition described in left panel. Right panel: Comparative HIV-specific CD4⁺ activation by each cell condition described in left panel. Experiments were performed in triplicates and in three different target-effector ratios. Panels show one representative experiment out of three that yielded similar results. Data show mean values and SEM. HLA: human leukocyte antigen; LPS: lipopolysaccharide; ITIP: IL-1β, TNF-α, IL-6 and PGE₂

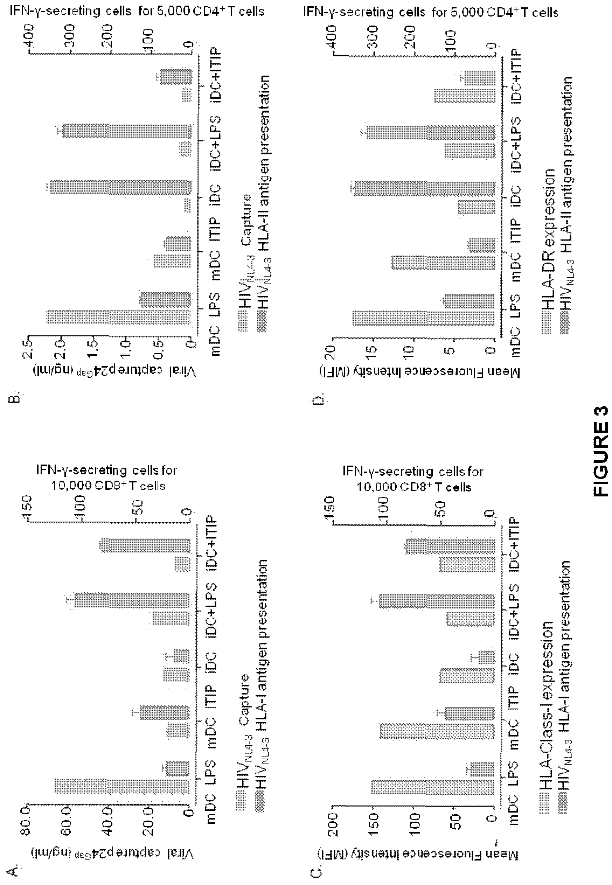

**Figure 3****.** Comparative analysis of the interferon gamma (IFN-γ) production by the EM40-F21 CD8⁺ T cell clone (A) and the N2 CD4⁺ T cell clone (B) in antigen presentation assays and the intracellular p24^{Gag} HIV_{NL4-3} content in each dendritic cell (DC) condition tested in Figure 2 before launching the ELISPOT assay. Relationship between the IFN-γ production by the EM40-F21 CD8⁺ T cell clone (C) and the N2 CD4⁺ T cell clone (D) in antigen presentation assays and the HLA-Class-I (C) and HLA-DR (D) expression in each DC condition tested in Figure 2 before launching the ELISPOT assay. Experiments were performed in triplicates for HIV_{NL4-3} and in three different target-effector ratios. Panels show one representative experiment out of three that yielded similar results. Data of IFN-γ secretion were taken from Figure 2 and show mean values and SEM. Similar results were obtained for HIV_{NL4-3ΔIN} (data not shown). HLA: human leukocyte antigen.

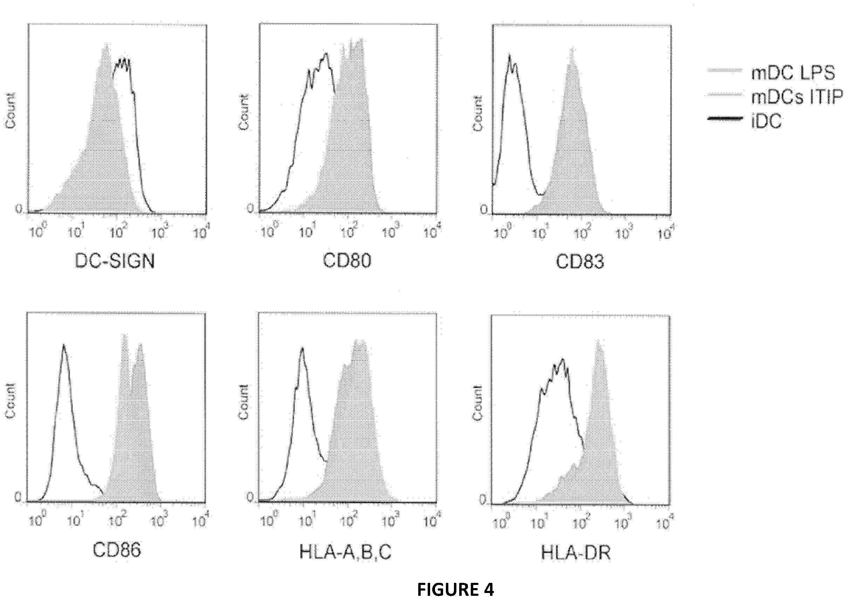

**Figure 4****.** Immunophenotyping of immature dendritic cells (iDC) and fully mature dendritic cells matured for 48 h with ITIP (mDCs ITIP) or with LPS (mDC LPS). Both maturation stimuli conferred a matured phenotype to DC, up-regulating HLA Class-I and Class-II molecules (HLA-A, B, C, HLA-DR) at cell surface as well as co-stimulatory molecules (CD80, CD83, CD86). LPS: lipopolysaccharide; ITIP: IL-1β, TNF-α, IL-6 and PGE₂.

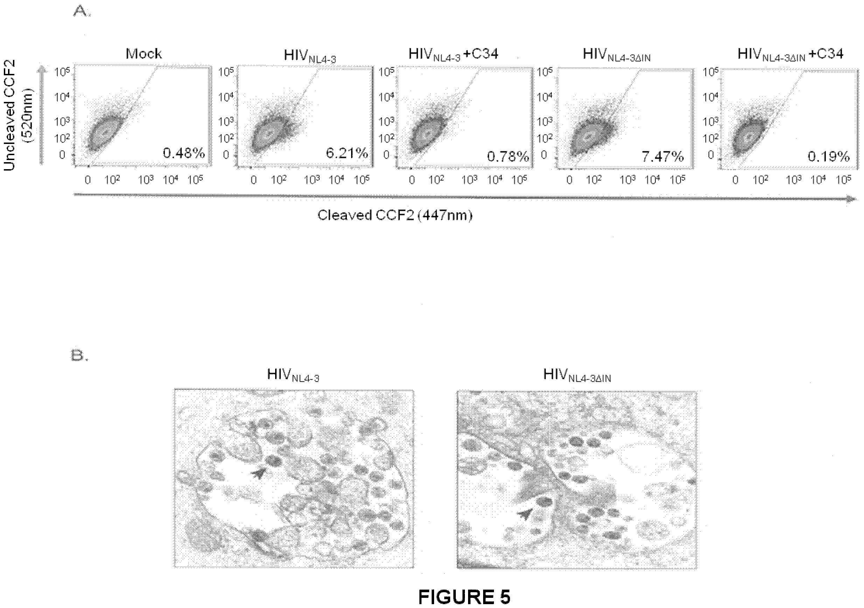

**Figure 5**. **(A)** Viral fusion assay of HIV_{NL4-3} and HIV_{NL4-3ΔIN} in the Jurkat T cell line using HIV-1 virions containing a β-lactamase-Vpr chimeric protein. Experiments were performed in the presence or absence of C34 fusion inhibitor. **(B)** Comparative electron microscopy images of fully mature dendritic cells cultured with LPS (mDC LPS) exposed to HIV_{NL4-3} (left panel) or HIV_{NL4-3ΔIN} (right panel), showing similar large vesicle location. Stars indicate captured particles, with the characteristic electron-dense structure of HIV-1 core.

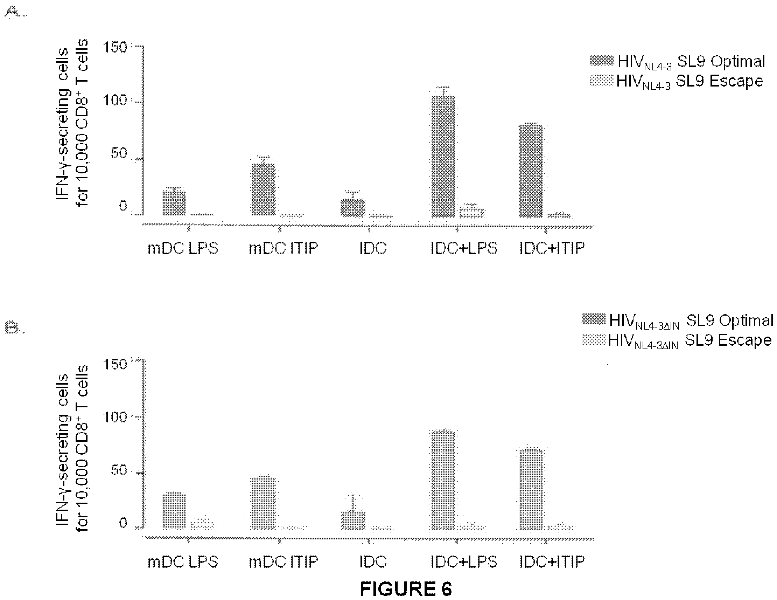

**Figure 6****.** Comparative analysis of the interferon gamma (IFN-γ) production by EM40-F21 CD8⁺ T cell clone when stimulated with dendritic cells (DC) pulsed with HIV_{NL4-3} **(A)** or HIV_{NL4-3ΔIN} **(B)** expressing the optimal (SLYNTVATL) or the escape (SLFNTIAVL) variant of SL9 epitope.

### DETAILED DESCRIPTION OF THE INVENTION

The present invention relates to a method for obtaining an antigen-loaded antigen-presenting cell based on loading an immature dendritic cell with an immunogen, preferably a HIV-1 integrase-deficient immunogen. More preferably, said cell is simultaneously placed under conditions adequate for maturation. This method allows obtaining antigen-presenting cells having higher antigen presentation abilities than those cells obtained when the antigen is loaded on fully matured dendritic cells. Figure 2 shows that the antigen-presenting cells of the invention produce a higher activation of HIV-specific CD8⁺ T cells or HIV-specific CD4⁺ T cells than those antigen-presenting cells obtained by other methods.

The present invention relates also to a HIV-1 integrase-deficient immunogen to load dendritic cells. This immunogen is safer than other known HIV-1 immunogens, such as a whole inactivated HIV-1. The use of HIV inactivated vaccines has been limited by safety concerns about potential residual infectivity in the product due to incomplete inactivation. However, the HIV-1 integrase-deficient immunogens of the invention have the advantage that they are unable to replicate. Examples of the present invention show that the HIV-1 integrase-deficient immunogen HIV_{NL4-3ΔIN} is captured by DCs and presented efficiently to CD4⁺ and CD8⁺ T cells, with the added advantage that no viral dissemination occurs.

### 1. Definitions of general terms αnd expressions

The term "AIDS", as used herein, refers to the symptomatic phase of HIV infection, and includes both Acquired Immune Deficiency Syndrome (commonly known as AIDS) and "ARC," or AIDS-Related Complex. *See* Adler M, et al., Brit. Med. J. 1987; 294: 1145-1147. The immunological and clinical manifestations of AIDS are well known in the art and include, for example, opportunistic infections and cancers resulting from immune deficiency.

The term "agonist of the IL-1 receptor", as used herein, refers to a cytokine that acts as an agonist of the interleukin-1 receptor (IL-1R). Agonists of the IL-1 receptor include, without limitation, IL-1α and IL-1β.

The term "antigen", as used herein, refers to any molecule or molecular fragment that, when introduced into the body, induces a specific immune response (i.e. humoral or cellular) by the immune system. Antigens have the ability to be bound at the antigen-binding site of an antibody. Antigens are usually proteins or polysaccharides. Antigens suitable for the present invention are parts of bacteria, viruses, parasites and other microorganisms such as coats, capsules, cell walls, flagella, fimbriae and toxins. Examples of antigens according to the present invention include antigens from picornavirus, coronavirus, togavirus, flavirvirus, rhabdovirus, paramyxovirus, orthomyxovirus, bunyavirus, arenavirus, reovirus, retrovirus, papilomavirus, parvovirus, herpesvirus, poxvirus, hepadnavirus, and spongiform virus families; or from other pathogens such as trypanosomes, tapeworms, roundworms, helminthes or malaria. Examples of suitable viral antigens are, without limitation: retroviral antigens from the human immunodeficiency virus (HIV) including gene products of the *gag, pol, env* and *nef* genes, and other HIV components; hepatitis viral antigens, such as the S, M, and L proteins of hepatitis B virus, the pre-S antigen of hepatitis B virus, and other hepatitis (e.g. hepatitis A, B, and C, viral components such as hepatitis C viral RNA); influenza viral antigens, such as hemagglutinin and neuraminidase and other influenza viral components; measles viral antigens, such as the measles virus fusion protein and other measles virus components; rubella viral antigens, such as proteins E1 and E2 and other rubella virus components; rotaviral antigens, such as VP7sc and other rotaviral components; cytomegaloviral antigens, such as envelope glycoprotein B and other cytomegaloviral antigen components; respiratory syncytial viral antigens, such as the RSV fusion protein, the M2 protein and other respiratory syncytial viral antigen components; herpes simplex viral antigens, such as immediate early proteins, glycoprotein D, and other herpes simplex viral antigen components; *varicella zoster* viral antigens, such as gpI, gpII, and other *varicella zoster* viral antigen components; Japanese encephalitis viral antigens, such as proteins E, M-E, M-E-NSI, NSI, NS1-NS2A, 80 percent E, and other Japanese encephalitis viral antigen components; rabies viral antigens, such as rabies glycoprotein, rabies nucleoprotein and other rabies viral antigen components. *See* Fields B, Knipe D, Eds., "Fundamental Virology", 2nd Ed. (Raven Press, New York, NY, US, 1991) for additional examples of viral antigens.

The term "antigen-loaded antigen-presenting cell", as used herein, refers to a dendritic cell with the ability to present an antigen that has been processed by said cell.

The term "antiretroviral therapy" or "ART", as used herein, refers to the administration of one or more antiretroviral drugs to inhibit the replication of HIV. Typically, ART involves the administration of at least one antiretroviral agent (or, commonly, a cocktail of antiretrovirals) such as nucleoside reverse transcriptase inhibitor (e.g. zidovudine (AZT, lamivudine (3TC) and abacavir), non-nucleoside reverse transcriptase inhibitor (e.g. nevirapine and efavirenz), and protease inhibitor (e.g. indinavir, ritonavir and lopinavir). The term Highly Active Antiretroviral Therapy ("HAART") refers to treatment regimens designed to aggressively suppress viral replication and progress of HIV disease, usually consisting of three or more different drugs, such as for example, two nucleoside reverse transcriptase inhibitors and a protease inhibitor.

The term "autologous", as used herein, means that the donor and recipient of the HIV-1 viral particle and the dendritic cell is the same subject.

The term "cell", as used herein, is equivalent to "host cell" and is intended to refer to a cell into which a viral genome, a vector or a HIV-1 viral particle of the invention has been introduced. It should be understood that such terms refer not only to the particular subject cell but to the progeny, or potential progeny, of such a cell. Because certain modifications may occur in succeeding generations due to either mutation or environmental influences, such progeny may not, in fact, be identical to the parent cell, but can be still included within the scope of the term as used herein.

The term "comprising" or "comprises", as used herein, discloses also "consisting of" according to the generally accepted patent practice.

The expression "conditions adequate for maturation", as used herein, refers to culturing an immature dendritic cell under conditions suitable to achieve the maturation of said cell. Suitable conditions for maturation are well-known by the skilled in the art. Mature dendritic cells can be prepared (i.e. matured) by contacting the immature dendritic cells with effective amounts or concentration of a dendritic cell maturation agent. Dendritic cell maturation agents can include, for example, BCG, IFNγ, LPS, monophosphoryl lipid A (MPL), eritoran (CAS no. 185955-34-4), TNFα and their analogs. Effective amounts of BCG typically range from about 105 to 107 cfu per milliliter of tissue culture media. Effective amounts of IFNγ typically range from about 100-1000 U per milliliter of tissue culture media. Bacillus Calmette-Guerin (BCG) is an avirulent strain of *M. bovis.* As used herein, BCG refers to whole BCG as well as cell wall constituents, BCG-derived lipoarabidomannans, and other BCG components that are associated with induction of a type 2 immune response. BCG is optionally inactivated, such as heat-inactivated BCG, formalin-treated BCG, and the like. The immature DCs are typically contacted with effective amounts of BCG and IFNγ for about one hour to about 48 hours. Suitable culture media include AIM-V®, RPMI 1640, DMEM, or X-VIVO 15™. The tissue culture media can be supplemented with amino acids, vitamins, cytokines (e.g. GM-CSF), or divalent cations, to promote maturation of the cells. Typically about 500 units/ml of GM-CSF is used.

The expression "conditions adequate for processing of the immunogen and presentation by the antigen-presenting cell", as used herein, refers to the incubation of the dendritic cell in a suitable medium to allow the capture of the immunogen and the processing and presentation of said immunogen to other cells of the immune system.

The term "contacting", as used herein, refers to the incubation of an immature dendritic cell in the presence of the immunogen destined for loading into the dendritic cell. Immature DCs are capable of capturing and internalizing said immunogens thus becoming an antigen-loaded dendritic cell (also named antigen-pulsed dendritic cell). Antigen capture by immature DCs is mediated by macropinocytosis, receptor-mediated antigen capture and engulfment of apoptotic bodies. Preferably, said incubation is performed at 37°C for 6 hours. The success of the antigen-loading step or pulse can be assayed by washing the pulsed dendritic cells to remove uncaptured immunogens and lysing said pulsed dendritic cells to measure the intracellular antigen content by an ELISA assay. For example, when the immunogen is a HIV viral particle, the intracellular content in p24^{Gag} antigen, present on the capsid surface of the viral particles, can be assayed.

The term "dendritic cell", as used herein, refers to any member of a diverse population of morphologically similar cell types found in lymphoid or non-lymphoid tissues. Dendritic cells are a class of "professional" antigen presenting cells, and have a high capacity for sensitizing HLA-restricted T cells. Specifically, the dendritic cells include, for example, lymphocytic dendritic cells (including cells which induce Th2 or immune tolerance), bone marrow dendritic cells (generally used dendritic cells, including immature and mature dendritic cells), Langerhans cells (dendritic cells important as antigen-presenting cells in the skin), interdigitating cells (distributed in the lymph nodes and spleen T cell region, and believed to function in antigen presentation to T cells), and follicular dendritic cells (important as antigen-presenting cells for B cells). Dendritic cells may be recognized by function, or by phenotype, particularly by cell surface phenotype. These cells are characterized by their distinctive morphology (having veil-like projections on the cell surface), intermediate to high levels of surface HLA-class II expression and ability to present antigen to T cells, particularly to naive T cells. *See* Steinman R, et al., Ann. Rev. Immunol. 1991; 9:271-196. The cell surface of dendritic cells is characterized by the expression of the cell surface markers CD1a+, CD4+, CD86+, or HLA-DR+.

The term "dendritic cell maturation agent", as used herein, refers to a compound capable of producing the maturation of the dendritic cell when the dendritic cell is incubated with said compound.

The term "dendritic cell precursor", as used herein, refers to any cell capable of differentiating into an immature dendritic cell in the presence of an appropriate cytokine (i.e. G-CSF, GM-CSF, TNFα, IL-4, IL-13, SCF (c-kit ligand), Flt-3 ligand, or a combination thereof). Examples of dendritic precursor cells include, but are not limited to, myeloid dendritic precursor cells, lymphoid dendritic precursor cells and plasmacytoid dendritic precursor cells. Phenotypic surface markers expressed by various subsets of dendritic precursor cells are well known in the art and may be used for the purpose of identification, for example, by flow cytometry or using immunohistochemical techniques.

The expression "disease associated with a HIV infection", as used herein, includes a state in which the subject has developed AIDS, but also includes a state in which the subject infected with HIV has not shown any sign or symptom of the disease.

The expression "disease which requires an immune response against the antigen which is loaded in the antigen-presenting cell", as used herein, refers to any disease susceptible of being prevented or treated with the administration of an antigen. Suitable diseases include, without limitation, infectious diseases (e.g. HIV) and cancer.

The term *"gag-pol* gene", as used herein, refers to a gene (Gene ID: 155348) that codes for the Gag and Pol polyproteins. The Gag polyprotein is processed during maturation to MA (matrix protein, p17); CA (capsid protein, p24); SP1 (spacer peptide 1, p2); NC (nucleocapsid protein, p7); SP2 (spacer peptide 2, p1) and p6. The Pol polyprotein is processed during maturation to reverse transcriptase, integrase, and HIV protease. Sequences of *gag* and *pol* genes found in HIV isolates can be readily found (e.g. GenBank and Los Alamos HIV sequence databases).

The term "gp130 utilizing cytokine", as used herein, refer to a cytokine that signal through receptors containing gp130. The signal-transducing component glycoprotein 130 (gp130), also called CD130, is a transmembrane protein that forms one subunit of type I cytokine receptors within the IL-6 receptor family. The gp130 utilizing cytokines (also known as IL-6-like cytokines) useful in the present invention include, interleukin 6 (IL-6), interleukin 11 (IL-11), interleukin 27 (IL-27), ciliary neurotrophic factor (CNTF), cardiotrophin-1 (CT-1), cardiotrophin-like cytokine (CLC), leukemia inhibitory factor (LIF), oncostatin M (OSM) and Kaposi's sarcoma-associated herpesvirus interleukin 6 like protein (KSHV-IL6).

The term "HIV immunogen", as used herein, refers to a protein or peptide antigen derived from HIV that is capable of generating an immune response in a subject and also refers to a HIV viral particle, being said particle a whole viral particle or a viral particle lacking one or more viral components but retaining the ability to generate an immune response. HIV immunogens for use according to the present invention may be selected from any HIV isolate (e.g. any primary or cultured HIV- 1, HIV-2, or HIV-3 isolate, strain, or clade). HIV isolates are now classified into discrete genetic subtypes. HIV-1 is known to comprise at least ten subtypes (A1, A2, A3, A4, B, C, D, E, PL F2, G, H, J and K). *See* Taylor B, et al., New Engl. J. Med 2008; 359(18):1965-1966. HIV-2 is known to include at least five subtypes (A, B, C, D, and E). Subtype B has been associated with the HIV epidemic in homosexual men and intravenous drug users worldwide. Most HIV-1 immunogens, laboratory adapted isolates, reagents and mapped epitopes belong to subtype B. In sub-Saharan Africa, India, and China, areas where the incidence of new HIV infections is high, HIV-1 subtype B accounts for only a small minority of infections, and subtype HIV-1 C appears to be the most common infecting subtype. Thus, in certain embodiments, it may be preferable to select immunogens from particular subtypes (e.g. HIV- 1 subtypes B or C). It may be desirable to include immunogens from multiple HIV subtypes (e.g. HIV-1 subtypes B and C, HIV-2 subtypes A and B, or a combination of HIV- 1, HIV-2, or HIV-3 subtypes) in a single immunological composition.

The term "HIV-1 viral genome", as used herein, refers to the entirety of the HIV-1 virus hereditary information. The HIV-1 viral genome is flanked by two LTRs and is composed of two copies of positive single-stranded RNA that encodes the virus nine genes. The HIV-1 RNA genome consists of at least seven structural landmarks (LTR, TAR, RRE, PE, SLIP, CRS, and INS), and nine genes (*gag, pol, env, tat, rev, nef, vif, vpr, vpu,* and sometimes a tenth *tev),* encoding 19 proteins. The HIV-1 viral genome comprises the following genes:
1) *gag* (group-specific antigen): codes for the Gag polyprotein, which is processed during maturation to MA (matrix protein, p17); CA (capsid protein, p24); SP1 (spacer peptide 1, p2); NC (nucleocapsid protein, p7); SP2 (spacer peptide 2, p1) and p6.
2) *pol:* codes for viral enzymes reverse transcriptase, integrase, and HIV protease.
3) *env* (for "envelope"): codes for gp160, the precursor to gp120 and gp41, proteins embedded in the viral envelope which enable the virus to attach to and fuse with target cells.
4) Transactivators: *tat* (for trans-activator), *rev* (for regulator of expression of virion protein), *vpr* (for viral protein R).
5) Other regulators: *vif* (for virion infectivity), *nef* (for negative factor), vpu (for viral protein U).
6) *tev:* this gene is only present in a few HIV-1 isolates. It is a fusion of parts of the *tat, env* and *rev* genes, and codes for a protein with some of the properties of tat, but little or none of the properties of *rev.*

The term "HIV-1 viral particle", as used herein, refers to a roughly spherical structure with a diameter of about 120 nm composed of two copies of positive single-stranded RNA that encodes the virus nine genes enclosed by a conical capsid composed of 2,000 copies of the viral protein p24. The single-stranded RNA is tightly bound to nucleocapsid proteins, p7, and enzymes needed for the development of the virion such as reverse transcriptase, proteases, ribonuclease and integrase. A matrix composed of the viral protein p17 surrounds the capsid ensuring the integrity of the virion particle. This is, in turn, surrounded by the viral envelope that is composed of two layers of fatty molecules called phospholipids taken from the membrane of a human cell when a newly formed virus particle buds from the cell. Embedded in the viral envelope are proteins from the host cell and about 70 copies of a complex HIV protein that protrudes through the surface of the virus particle. This protein, known as Env, consists of a cap made of three molecules called glycoprotein (gp) 120, and a stem consisting of three gp41 molecules that anchor the structure into the viral envelope. This glycoprotein complex enables the virus to attach to and fuse with target cells to initiate the infectious cycle.

The term "human immunodeficiency virus" or "HIV", as used herein is meant to include HIV-1 and HIV-2. "HIV- 1" means the human immunodeficiency virus type-1. HIV-1 includes, but is not limited to, extracellular virus particles and HIV-1 forms associated with HIV-1 infected cells. "HIV-2" means the human immunodeficiency virus type-2. HIV-2 includes, but is not limited to, extracellular virus particles and HIV-2 forms associated with HIV-2 infected cells. Preferably, HIV is HIV-1.

The term "immature dendritic cell", as used herein, refers to a dendritic cell having significantly low T cell-activating ability as compared with a dendritic cell in a matured state. Specifically, the immature dendritic cells may have an antigen-presenting ability that is lower than 1/2, preferably lower than 1/4 of that of dendritic cells which maturation had been induced by adding LPS (1 µg/ml) and culturing for two days. The antigen-presenting ability can be quantified, for example, using the allo T cell-activating ability (mixed lymphocyte test: allo T cells and dendritic cells are co-cultured at a T cell:dendritic cell ratio of 1:10, or preferably at varied ratios; 3H-thymidine is added 8 hours before terminating cultivation, and the T cell growth capacity is assessed based on the amount of 3H-thymidine incorporated into the DNA of the T cells. *See* Jonuleit H, et al., Gene Ther. 2000; 7:249-254. Alternatively, it can be assessed by testing the ability to induce specific cytotoxic T cells (CTLs) using a peptide, in which a known class **I-**restricted peptide of a certain antigen is added to dendritic cells; the dendritic cells are co-cultured with T cells obtained from peripheral blood of the same healthy donor from whom the dendritic cells had been collected (with 25 U/ml or preferably 100 U/ml of IL-2 on day 3 or later). The T cells are preferably stimulated with dendritic cells three times during 21 days, more preferably stimulated with dendritic cells twice during 14 days. The resulting effector cells are co-cultured for four hours with 51Cr-labeled target cells (peptide-restricted class I positive tumor cells) at a ratio of 100:1 to 2.5:1 (100:1, 50:1, 25:1, 20:1, 12.5:1, 10:1, 5:1, or 2.5:1), preferably at a ratio of 10:1; and 51Cr released from the target cells is quantified. *See* Hristov G, et al., Arch. Dermatol. Res. 2000; 292:325-332. Furthermore, the immature dendritic cells preferably have phagocytic ability for antigens, and more preferably show low (for example, significantly low as compared to mature DCs induced by LPS as described above) or negative expression of receptors that induce the co-stimulation for T cell activation. Immature dendritic cells express surface markers that can be used to identify such cells by flow citometry or immunohistochemical staining.

The term "immunogen", as used herein, refers to a substance that is able to provoke an adaptative immune response if injected on its own. All immunogens are also antigens but not all antigens are immunogens.

The term "immunogenic composition", as used herein, refers to a composition that elicits an immune response in a subject that produces antibodies or cell-mediated immune responses against a specific immunogen. Immunogenic compositions can be prepared, for instance, as injectables such as liquid solutions, suspensions, and emulsions. The term "antigenic composition" refers to a composition that can be recognized by a host immune system. For example, an antigenic composition contains epitopes that can be recognized by humoral or cellular components of a host immune system.

The term "incubation", as used herein, refers to maintaining the culture of the dendritic cells in a maturation medium during a specific time, preferably during 48 hours, until the immature dendritic cell is transformed in a mature dendritic cell. The term "medium" is maturation substrate comprising a suitable culture media, one or more maturation agents and, optionally, other supplements.

The term "integrase protein" or "IN", as used herein, refers to the gene product of the *int* region of a virus, particularly of the HIV-1 virus, characterized by having three clearly identifiable domains: the central catalytic core domain flanked by the N-terminal and C-terminal domains, the latter involved in DNA binding. A single polypeptide chain of most retroviral INs comprises approximately 290 residues. Some important variations are, however, present. For example, PFV IN is significantly longer, comprising 392 residues, and ASV IN is encoded as a 323-amino acid long protein that is posttranslationally processed to the final polypeptide consisting of 286 residues, which is fully enzimatically active. Particularly, the integrase of HIV-1 virus is the gene product of the *int* region of the virus, having 288 amino acids and designated as IN. The term "integrase", as used herein, refers to the mature processed form of the HIV-1 integrase polypeptide defined under accession number C7B8I1 in the Uniprot database (release December 14^{th}, 2011. Version 18). The term HIV-1 integrase is also used to refer to HIV-1 integrase from other strains or isolates of the virus. The nucleic acid and amino acid sequence of a large number of HIV-1 integrases are readily available to the public. *See* HIV Sequence Database, http://www.hiv.lanl.gov/content/sequence/HIV/mainpage.html, January 2012; Los Alamos HIV Databases and Compendia, http://www.hiv.lanl.gov/, January 2011.

The term "mature dendritic cell", as used herein, is a cell that has significantly strong antigen-presenting ability for T cell or the like as compared with a dendritic cell in the immature state. Specifically, the mature dendritic cells may have an antigen-presenting ability that is half or stronger, preferably equivalent to or stronger than the antigen-presenting ability of dendritic cells in which maturation has been induced by adding LPS (1 µg/ml) and culturing for two days. Mature DC display up-regulated expression of co-stimulatory cell surface molecules and secrete various cytokines. Specifically, mature DCs express higher levels of HLA class I and class II antigens (HLA-A, B, C, HLA-DR) and are generally positive for the expression of CD80, CD83 and CD86 surface markers.

The term "medicament", as used herein, is understood to be a pharmaceutical composition, particularly a vaccine, comprising the immunogenic composition of the invention.

The term "monocytic dendritic cell precursors" or MoDC precursors, as used herein, comprises monocytes that have the GM-CSF receptor on their surface and other myeloid precursor cells that are responsive to GM-CSF. The cells can be obtained from any tissue where they reside, particularly lymphoid tissues such as the spleen, bone marrow, lymph nodes and thymus. Monocytic dendritic cell precursors also can be isolated from the circulatory system. Peripheral blood is a readily accessible source of monocytic dendritic cell precursors. Umbilical cord blood is another source of monocytic dendritic cell precursors.

The term "operably linked", as used herein, means that the nucleotide sequence of interest is linked to the regulatory sequence(s) in a manner that allows for expression of the nucleotide sequence (e.g. in an *in vitro* transcription/translation system or in a host cell when the vector is introduced into the host cell). *See* Auer H, Nature Biotechnol. 2006; 24: 41-43.

The terms "pharmaceutically acceptable carrier," "pharmaceutically acceptable diluent," "pharmaceutically acceptable excipient", or "pharmaceutically acceptable vehicle", used interchangeably herein, refer to a non-toxic solid, semisolid or liquid filler, diluent, encapsulating material or formulation auxiliary of any conventional type. A pharmaceutically acceptable carrier is essentially non-toxic to recipients at the employed dosages and concentrations and is compatible with other ingredients of the formulation. The number and the nature of the pharmaceutically acceptable carriers depend on the desired administration form. The pharmaceutically acceptable carriers are known and may be prepared by methods well known in the art. *See* Faulí i Trillo C, "Tratado de Farmacia Galénica" (Ed. Luzán 5, S.A., Madrid, ES, 1993) and Gennaro A, Ed., "Remington: The Science and Practice of Pharmacy" 20th ed. (Lippincott Williams & Wilkins, Philadelphia, PA, US, 2003).

The term "prevention", as used herein, means the administration of an immunogenic composition of the invention or of a medicament containing it in an initial or early stage of the infection, to avoid the appearance of clinical signs.

The expression "pro-inflammatory cytokine cocktail", as used herein, refers to a mixture of two or more cytokines that are able to trigger the maturation of immature dendritic cells. Examples of such cytokines are, without limitation, IL-1β, IL-6, TNF-α, IL-18, IL-11, IL-27, and IFN-α. Suitable pro-inflammatory cytokine cocktails are, without limitation, a cocktail formed by TNF-α and CD40L; a cocktail formed by IFN-α and TNF-α; a cocktail formed by IFN-α and CD40L; a cocktail formed by IFN-α, TNF-α and CD40L; a cocktail formed by TNF-α, IL-1β and IL-6; a cocktail formed by IL-1β, TNF-α, IFN-α, IFN-γ and poly (I:C); a cocktail formed by IL-1, IL-6, TNF-α, IFN-α, and CD40L.

The term "prostaglandin", as used herein, refers to a member of a group of lipid compounds that are derived enzymatically from fatty acids and have important functions in the animal body. Every prostaglandin contains 20 carbon atoms, including a 5-carbon ring. Examples of prostaglandins useful in the present invention are, without limitation, prostacyclin I₂ (PGI₂), prostaglandin E₂ (PGE₂) and prostaglandin F_{2α} (PGF_{2α}).

The term "TLR4 ligand" or "toll-like receptor 4 ligand", as used herein, refers to a ligand of the toll-like receptor 4 (TLR4). TLR4 has also been designated as CD284 (cluster of differentiation 284) and is a member of the Toll-like receptor family, which plays a fundamental role in pathogen recognition and activation of the innate immune system.

The term "TNF superfamily member", as used herein, refers to a cytokine that pertains to the tumor necrosis factor (TNF) superfamily. The TNF superfamily of cytokines represents a multifunctional group of pro-inflammatory cytokines which activate signaling pathways for cell survival, apoptosis, inflammatory responses and cell differentiation. Examples of TNF superfamily members include, without limitation, tumor necrosis factor alpha (TNFα), LIGHT, CD40 ligand (CD40L), 4-1BB ligand (4-1BBL), APRIL, CD27 ligand (CD27L), CD30 ligand (CD30L), Fas ligand, glucocorticoid-induced TNFR-related ligand (GITRL), lymphotoxin alpha (LTα), lymphotoxin beta (LTβ), OX40 ligand (OX40L), receptor activator of NF- κB ligand (RANKL), B cell-activating factor of the TNF family (BAFF), TNF-related apoptosis-inducing ligand (TRAIL), TNF-like weak inducer of apoptosis (TWEAK) and VEG1.

The term "treat" or "treatment", as used herein, refers to the administration of an immunogenic composition of the invention or of a medicament containing it to control the progression of the disease before or after clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival, compared with the expected survival if treatment was not applied. Within the context of the present invention, the terms "treat" and "treatment" refer specifically to preventing or slowing the infection and destruction of healthy CD4+ T cells in a HIV-1 infected subject. It also refers to the prevention and slowing the onset of symptoms of the acquired immunodeficiency disease such as extreme low CD4+ T cell count and repeated infections by opportunistic pathogens such as *Mycobacteriα spp., Pneumocystis carinii,* and *Pneumocystis cryptococcus.* Beneficial or desired clinical results include, but are not limited to, an increase in absolute naive CD4+ T cell count (range 10-3520), an increase in the percentage of CD4+ T cell over total circulating immune cells (range 1-50 percent), and/or an increase in CD4+ T cell count as a percentage of normal CD4+ T cell count in an uninfected subject (range 1-161 percent). "Treatment" can also mean prolonging survival of the infected subject as compared to expected survival if the subject did not receive any HIV targeted treatment.

The term "vaccine", as used herein, refers to an immunogenic composition for *in vivo* administration to a host, which may be a primate, especially a human host, to confer protection against a disease, particularly a viral disease.

The term "vector", as used herein, denotes a nucleic acid molecule, linear or circular, that comprises the genome encoding all the proteins forming a viral particle (except a part or the total of the integrase protein) operably linked to additional segments that provide for its autonomous replication in a host cell of interest. Preferably, the vector is an expression vector, which is defined as a vector, which in addition to the regions of the autonomous replication in a host cell, contains regions operably linked to the genome of the invention and which are capable of enhancing the expression of the products of the genome according to the invention.

The term "viral particle", as used herein, refers to a whole viral particle and not to a protein subunit or peptide. Viral particles (also known as virions) consist of two or three parts: the genetic material of the virus made from either DNA or RNA; a protein coat that protects these genes; and, in some cases, an envelope of lipids that surrounds the protein coat when they are outside a cell. The shape of the viral particle ranges from simple helical and icosahedral forms to more complex structures, depending on the virus.

### 2. Method for obtaining an antigen-loaded antigen-presenting cell in vitro

In a first aspect, the invention relates to a method (hereinafter referred to as "first method of the invention") for obtaining an antigen-loaded antigen-presenting cell *in vitro* comprising contacting an immature dendritic cell with an immunogen under conditions adequate for its processing and presentation by the antigen-presenting cell.

In a preferred embodiment, the immunogen is a viral particle, preferably a HIV viral particle, more preferably a HIV-1 viral particle. The viral particle may contain several antigens.

The HIV-1 virus exhibits an unusually high degree of genetic variability throughout its genome. Sequence comparisons have identified three genetic groups of HIV-1, designated M, O, and N. The existence of a fourth group, "P", has been hypothesized based on a virus isolated in 2009. Group M is further divided into phylogenically related major genetic subtypes (or clades), designated A, B, C, D, E, F, G, H, J and K. Co-infection with distinct subtypes gives rise to circulating recombinant forms (CRFs). Together with circulating inter-subtype recombinant forms (CRFs), group M comprises the majority of HIV-1 variants in the world today. The HIV-1 virus of the present invention may represent any of the genetic groups or genetic subtypes capable of infecting a human being, and also includes circulating recombinant forms, laboratory strains and primary isolates. Thus, in a preferred embodiment the immunogen is a HIV immunogen.

Suitable HIV immunogens include the HIV envelope (env; e.g. NCBI Ref. Seq. NPJ357856), gag (e.g. p6, p7, p17, p24, GenBank AAD39400.1), the protease encoded by pol (e.g. UniProt P03366), nef (e.g. GenBank CAA41585.1, Shugars D, et al., J. Virol. 1993; 67(8):4639-4650), as well as variants, derivatives, and fusion proteins thereof. *See* Gómez C, et al., Vaccine 2007; 25:1969-1992. Suitable strains and combinations may be selected by the skilled artisan as desired.

The HIV immunogen of the invention is capable of eliciting an immune response. Particularly, "immune response" refers to a CD4+ T cell or CD8+ T cell mediated immune response to HIV infection. An immune response to HIV may be determined by measuring, for example, viral load, T cell proliferation, T cell survival, cytokine secretion by T cells, or an increase in the production of antigen-specific antibodies (e.g. antibody concentration).

Dendritic cell therapy represents a new and promising approach for the treatment or prevention of many diseases such as cancer or HIV infection. Said therapies comprise the use of dendritic cells containing one or more antigens that have been introduced artificially into said cells to obtain "antigen-loaded dendritic cells".

The method of the invention comprises contacting an immature dendritic cell with an immunogen under conditions adequate for its processing and presentation by an antigen-presenting cell.

The dendritic cells affected by the methods of the invention may be selected to be immature or mature dendritic cells. Immature dendritic cells can be obtained from a population of dendritic cell precursors. Preferably, the dendritic cell precursor is a cell that can differentiate into an immature dendritic cell in four weeks or less, more preferably, in 20 days or less, even more preferably, in 18 days or less, and still more preferably, in 16 days or less. In a preferred embodiment, the dendritic cell precursor differentiates into an immature dendritic cell in the presence of GM-CSF and IL-4 in less than seven days, and more preferably, in five days.

In a preferred embodiment, the population of dendritic precursor cells is a population of monocytic dendritic cell precursors. More preferably, the monocytic dendritic cell precursors are derived from peripheral blood mononuclear cells (PBMCs). The PBMCs can be obtained either from whole blood diluted 1:1 with buffered saline or from leukocyte concentrates ("buffy coat" fractions, MSKCC Blood Bank) by standard centrifugation over Ficoll-Paque PLUS (endotoxin-free, catalog no. 17-1440-03, Amersham Pharmacia Biotech AB, Uppsala, SE). MoDC precursors are tissue culture plastic-adherent (catalog no. 35-3003, Falcon, Becton-Dickinson Labware Inc., Franklin Lakes, NJ, US) PBMCs, and can be cultured in complete RPMI 1640 plus 1% normal human serum (NHS) (or 10% fetal bovine serum) in the presence of GM-CSF (1000 IU/ml) and IL-4 (500 IU/ml) with replacement every 2 days as described. *See* Thurner B, et al., J. Immunol. Meth. 1999; 223:1-15 and Ratzinger G, et al., J. Immunol. 2004; 173:2780-2791.

Purified monocyte populations can be isolated from PBMCs with CD14⁺ antibodies prior to the culture to obtain immature dendritic cells. Monocytes are usually identified in stained smears by their large bilobate nucleus. In addition to the expression of CD14, monocytes express also, among others, one or more of the following surface markers: 125I-WVH-1, 63D3, adipophilin, CB 12, CDl Ia, CDl Ib, CD15, CD54, Cd163, cytidine deaminase, and FIt-I. *See* Feyle D, et al., Eur. J. Biochem. 1985; 147:409-419, Malavasi F, et al., Cell Immunol. 1986; 97(2):276-285, Rupert J, et al., Immunobiol. 1991; 182(5):449-464; Ziegler-Heitbrock H, J. Leukoc. Biol. 2000; 67:603-606, and Pilling D, et al., PLoS One 2009; 4(10):e-7475.

In general, monocytic dendritic cell precursors may be identified by the expression of markers such as CD13 and CD33. Myeloid dendritic precursors may differentiate into dendritic cells via CD14 or CD1a pathways. Accordingly, a dendritic precursor cell of the invention may be a CD14+CD1a- dendritic precursor cell or a CD14-CD1a+ dendritic precursor cell. In certain embodiments of the invention, a myeloid dendritic precursor cell may be characterized by the expression of SCA-1, c-kit, CD34, CD16, and CD14 markers. In a preferred embodiment, the myeloid dendritic precursor cell is a CD14+ monocyte. The CD14+ monocyte may also express the GM-CSF receptor.

The immature dendritic cells used as starting material for the method of the invention can be autologous to the subject to be treated. In other embodiments, the immature dendritic cells used as starting material for the methods of the invention are heterologous dendritic cells. For example, if graft-versus-host disease is to be treated, the immature dendritic cells that are being used as starting material are dendritic cells that were obtained from the donor. The subj ect can be, for instance, a mouse, a rat, a dog, a chicken, a horse, a goat, a donkey, or a primate. Most preferably, the subject is a human. In a preferred embodiment, the immature dendritic cell is a monocyte-derived immature dendritic cell.

The first method of the invention comprises contacting said immature dendritic cells with an immunogen comprising the antigen to be introduced in the dendritic cell under conditions adequate for processing of the immunogen and presentation by the antigen-presenting cell. As a result, an antigen-loaded antigen-presenting cell is obtained.

In one embodiment, the first method of the invention further comprises placing the immature dendritic cell under conditions adequate for its maturation wherein said maturation step is carried out simultaneously with the step of contacting the immature dendritic cell with an immunogen. In another embodiment, the immature dendritic cell is first loaded with the immunogen and matured afterwards. In yet another embodiment, the dendritic cell is first matured and is then loaded with the immunogen. Preferably, the immature cell is matured while it is being loaded with the immunogen.

The second step of the first method of the invention comprises placing said immature dendritic cell under conditions adequate for its maturation into a mature dendritic cell. In a preferred embodiment, the conditions adequate for maturation comprise the incubation of the immature dendritic cells in a medium comprising a dendritic cell maturation agent selected from the group consisting of:
i) a TLR4 ligand and
ii) a pro-inflammatory cytokine cocktail.

In a preferred embodiment, the TLR4 ligand for use in the method according to the present invention include, without limitation, LPS, MPL, heat shock protein 60 (Hsp60), heat shock protein 70 (Hsp70), heat shock protein gp96, fibrinogen, heparan sulfate, soluble hyaluronan, beta-defensin 2 and angelan. *See* Kim J, et al. Cancer Letters 2011; 313(2): 226-234. More preferably, the TLR4 ligand is LPS. *See* Castiello L, et al., Cancer Immunol. Immunother. 2011; 60(4): 457-466. Preferably, the incubation with LPS is performed at a 100 ng/mL concentration.

In an attempt to recreate a physiological environment for DC maturation, some balanced cocktails of maturation agents have been used. Thus, in another preferred embodiment, the cell maturation agent is a pro-inflammatory cytokine cocktail. In a preferred embodiment, the pro-inflammatory cytokine cocktail comprises at least an agonist of the IL-1 receptor, a gap 130 utilizing cytokine and a TNF superfamily member. Said cytokine cocktail can include other compounds.

In a preferred embodiment, the IL-1 receptor agonist is IL-1β. Preferably, the effective IL-1β concentration is 300 U/mL. In another preferred embodiment, the gp130 utilizing cytokine is IL-6. Preferably, the effective IL-6 concentration is 1000 U/mL of IL-6. In another preferred embodiment, the TNF superfamily member is TNFα. Preferably, the effective TNFα concentration is 1000 U/mL.

The most frequently used cocktail contains TNF-α, IL-1β and IL-6. Thus, in a preferred embodiment the pro-inflammatory cytokine cocktail comprises a mixture of It-1β, IL-6 and TNF-α. More preferably, the composition of the medium is 300 U/mL of IL-1β, 1000 U/mL of TNF-α and 1000 U/mL of IL-6.

It has been disclosed that the addition of a prostaglandin to the pro-inflammatory cytokine cocktail improves the yield, maturation, migratory and immunostimulatory capacity of the DC generated. *See* Jonuleit H, et al., Eur. J. Immunol. 1997; 27: 3135-3142. Thus, in a preferred embodiment the pro-inflammatory cytokine cocktail further comprises a prostaglandin. More preferably, the prostaglandin utilized is prostaglandin E₂ (PGE₂). Preferably, the effective PGE₂ concentration is 1 µg/mL. More preferably, the composition of the medium is 300 U/mL of IL-1β, 1000 U/mL of TNF-α, 1000 U/mL of IL-6 and 1 µg/mL of PGE₂.

At the end of the incubation time a mature dendritic cell is obtained. Maturation of dendritic cells can be monitored by methods known in the art for dendritic cells. mDCs surface markers can be detected in assays such as flow cytometry and immunohistochemical staining. The mDCs can also be monitored by cytokine production (e.g. by ELISA, another immune assay, or by use of an oligonucleotide array). The maturation of a dendritic cell can be further confirmed by immunophenotyping. An immature dendritic cell may be distinguished from a mature dendritic cell, for example, based on markers selected from the group consisting of CD80 and CD86. An immature dendritic cell is weakly positive and preferably negative for these markers, while a mature dendritic cell is positive.

When the method of the invention takes place in a culture having a population of immature dendritic cells, conditions adequate for maturation are such where the maturation of at least 50%, at least 60%, at least 70%, at least 80%, at least 90%, or preferably 100% of immature dendritic cells is achieved.

The first method of the invention is characterized by the fact that both steps: i) the step of contacting the immature dendritic cell with an immunogen and ii) the step of maturing the immature dendritic cell, are carried out simultaneously during at least part of said steps i) or ii). Thus, both steps can overlap completely, when the maturation of the dendritic cell and the contact with the immunogen starts at the same time; or the dendritic cell may be incubated with the immunogen only during part of the maturation step, for example when the maturation of the dendritic cell starts first and after some time during the maturation step the dendritic cell is contacted with the immunogen; or the maturation may take place only during part of the incubation of the dendritic cell with the immunogen, for example if the incubation with the immunogen starts first and then, after some time, the maturation of the dendritic cell is started.

When steps i) and ii) of the first method of the invention have finished, a matured antigen-loaded dendritic cell is obtained (i.e. a mature dendritic cell carrying the antigen of interest). The maturation of the dendritic cells can be confirmed by immunophenotyping as previously indicated. The antigen-loading by the dendritic cells can be assayed by the methods described above.

In a preferred embodiment, the immunogen to be loaded into a dendritic cell is a viral particle, preferably, a retroviral viral particle. In another preferred embodiment, the immunogen is a lentivirus particle, preferably, a HIV viral particle. More preferably, the immunogen is a HIV-1 viral particle.

HIV-1 virus binds with and subsequently infects human CD4 cells through the use of a co-receptor on the cell surface. Different strains of HIV-1 use different coreceptors to enter human CD4 cells. Thus, HIV-1 virus can be CCR5-tropic when the virus strain only uses the C-C chemokine receptor type 5 (CCR5) co-receptor to infect CD4 cells; CXCR4-tropic when a virus strain only uses the C-X-C chemokine receptor type 4 (CXCR4) co-receptor to infect the CD4 cells; and dual-tropic when the virus strain can use either the CCR5 or CXCR4 co-receptor to infect CD4 cells. *See* Whitcomb J, et al., Antimicrob. Agents Chemother. 2007; 51(2):566-575. There are available several assays to distinguish between different tropic viruses (e.g. Trofile®, Monogram Biosciences, Inc., San Francisco, CA, US). In a preferred embodiment, the HIV-1 virus is selected from a CXCR4-tropic virus and a CCR5-tropic virus, preferably is a CXCR4-tropic virus.

Lentiviruses, including HIV, are transmitted as single-stranded, positive-sense, enveloped RNA viruses. Upon entry into the target cell, the viral RNA genome is converted (reverse transcribed) into double-stranded DNA by a virally encoded reverse transcriptase that is transported along with the viral genome in the virus particle. The resulting viral DNA is then imported into the cell nucleus and integrated into the cellular DNA by a virally encoded integrase and host co-factors. Once integrated, the virus may become latent, allowing the virus and its host cell to avoid detection by the immune system. Alternatively, the virus may be transcribed, producing new RNA genomes and viral proteins that are packaged and released from the cell as new virus particles that begin the replication cycle anew.

The present invention discloses that a viral particle lacking a functional integrase protein can be a good immunogen since the viral DNA cannot integrate into the host cellular DNA and thus cannot replicate. However, said viral particle lacking a functional integrase protein maintains the same structure of viral antigens of the wild-type virus and is thus capable of inducing a comparable immune response.

Integrase is a retrovirus-specific enzyme encoded by all retroviral genomes. Integrases for the purpose of this invention include, among others, integrases from HIV-1, HIV-2, avian sarcoma virus (ASV), simian immunodeficiency virus (SIV), human foamy virus (PFV), Mason-Pfizer monkey virus (MPMV) and feline immunodeficiency virus (FIV). Thus, in a preferred embodiment the immunogen is a viral particle lacking a functional integrase protein, preferably, a retroviral particle, and more preferably, a HIV-1 viral particle.

The HIV-1 viral particle of the invention lacks a functional integrase protein. The expression "lack of a functional integrase protein" includes not only the situation in which the integrase protein is not synthesized and the viral particle does not contain integrase but also includes situations in which the integrase protein is synthesized but is inactive or essentially inactive. An integrase protein inactive or essentially inactive is an integrase protein lacking at least 95%, 96%, 97%, 98%, 99%, preferably, 100% of its function (or activity).

Integrase activity can be assessed by measuring its residual activity. *See* Philippe S, et al., Proc. Natl. Acad. Sci. USA 2006; 103(47):17684-17689. Other indirect method to test integrase activity is to assay the replication capacity of the virus by determining the kinetics of p24Gag production in cells such as PBMCs by methods known in the art.

Viral particles lacking a functional integrase protein for use in the present invention can be defective in another protein or have some mutation or deletion in the genome encoding them with respect to a viral particle wild-type. However, in a more preferred embodiment the HIV-1 viral particle contains a full complement of all the viral genes except the *pol* gene.

The expression "contains a full complement of all the viral genes except the *pol* gene" means that all the viral genes are complete and unaltered except the *pol* gene, which is the gene that encodes for the integrase protein. For example, the viral particle of said embodiment may be a viral particle produced after genetic engineering of the integrase encoding region of the genome of a viral particle isolated from nature (e.g. from a sample of a patient).

The lack of a functional integrase protein of the viral particle of the invention may be due to different reasons including:
i) one or more natural or artificial site-specific mutations provoking a change in an amino acid by a different amino acid that produce a lack of the integrase function. In such case the integrase protein is present in the viral particle but is not functional.
ii) a deletion of one or more amino acids that produce a lack of the integrase function. In such case the integrase protein is present having a shorter size than the wild-type integrase protein but is not functional.
iii) a deletion of the complete region of the viral genome encoding the integrase protein. In such case the integrase protein is not present in the viral particle.

In a more preferred embodiment, the HIV-1 viral particle lacking a functional integrase protein contains a deletion in the integrase protein or lacks the integrase protein, preferably lacks the integrase protein.

The expression "deletion in the integrase protein" refers to a deletion in one or more amino acids of the protein thus producing a non-functional protein having a shorter size than the wild-type integrase protein (i.e. that the integrase polypeptide is not the full-length integrase protein). Preferably, the integrase protein lacks the central catalytic core domain or a part of said domain.

The expression "lacks the integrase protein" or "lacking the integrase protein", as used herein, means that the viral particle is not capable of synthesizing the integrase protein due to a complete deletion of the region of the gene that encodes the integrase protein.

In a second aspect, the invention relates to a method (hereinafter referred to as the "second method of the invention") for obtaining a dendritic cell loaded with a HIV-1 immunogen *in vitro* comprising loading a dendritic cell with a HIV-1 viral particle lacking functional integrase protein.

The HIV-1 viral particle lacking functional integrase protein of the second method of the invention may be a HIV-1 viral particle generated from a HIV-1 viral particle isolated from a HIV-1 infected subject.

The expression "generating a HIV-1 viral particle lacking a functional integrase protein" refers to the artificial production of a HIV-1 viral particle lacking a functional integrase protein by using genetic engineering techniques. The generation of said defective viral particle may be by site-directed mutagenesis or gene deletion techniques well known in the art. *See* Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

To quantify the reduction in the integrase activity produced by the deletion in the integrase encoding region, and to quantify the residual integrase activity that remains in the virus after said deletion, the HIV-1 viral particle lacking a functional integrase protein may be submitted to an assay such as previously described. *See* Philippe S, 2006, *supra.*

The expression "HIV-1 viral particle isolated from a HIV-1 infected subject" means that the particle lacking a functional integrase protein is obtained after genetic engineering manipulation of a HIV-1 viral genome obtained from a HIV-1 viral particle isolated from a HIV-1 infected subject.

The HIV-1 viral particle is isolated from the subject prior to the treatment to generate a HIV-1 integrase deficient immunogen. In the context of the second aspect of the invention, the term "isolation" or "isolated" refers to a HIV-1 viral particle separated from the blood of a donor subject. Typically, a HIV-1 viral particle can be isolated from CD4+ T lymphocytes by methods known in the art. Such cells may be isolated from a peripheral blood sample. Said peripheral blood sample is obtained under aseptic conditions and may contain an anticoagulant.

The expression "HIV-1 infected subject", as used herein, refers to a human subject infected by HIV-1. HIV-1 infected subjects are identified by clinical conditions associated with HIV infection and CD4+ T lymphocyte counts. From a practical standpoint, the clinician views HIV infection as a spectrum of disorders ranging from primary infection with or without the acute HIV syndrome to the symptomatic infection state of advanced disease. The Centers for Disease Control and Prevention (CDC) in Atlanta, Georgia, has established an authoritative definition for the diagnosis of AIDS, which informs a skilled artisan whether the onset of AIDS has occurred: in a HIV-infected subject, the CD4+ T cell count must be below 200 cells per cubic mm of blood, or there must be the clinical appearance of an initial AIDS-defining opportunistic infection, such as PCP (i.e. *Pneumocystis carinii* pneumonia), oral candidiasis (i.e. thrush), pulmonary tuberculosis, or invasive cervical carcinoma. Methods of determining a subject's CD4+ T cell count are known in the art. In a preferred embodiment, the subject has discontinued antiretroviral therapy.

The subject may be asymptomatic or may have already developed some of the signs associated with diseases resulting from HIV infection. Moreover, the subject may also be under treatment with a retroviral therapy (e.g. HAART therapy). In a preferred embodiment, the subject has at least 450 ×10⁶/L CD4+ T cells or more than 10,000 copies of HIV-1 RNA per mL of plasma.

The second method of the invention comprises loading a dendritic cell with a HIV-1 viral particle lacking a functional integrase protein. This step is carried out essentially as described above in the context of the first method of the invention.

The dendritic cells used in the second method of the invention are mature dendritic cells which have been obtained from immature dendritic cells using any of the methods described above for inducing maturation of the dendritic cells. In a preferred embodiment, the dendritic cells are obtained by maturation from immature dendritic cells using a medium comprising a dendritic cell maturation agent selected from the group consisting of:
i) a TLR4 ligand and
ii) a pro-inflammatory cytokine cocktail.

In a preferred embodiment, the TLR4 ligand for use in the method according to the present invention include LPS. In another embodiment, the the pro-inflammatory cytokine cocktail comprises at least an agonist of the IL-1 receptor, a gp130 utilizing cytokine and a TNF superfamily member. Said cytokine cocktail can include other compounds. In a still more preferred embodiment, the IL-1 receptor agonist is IL-1β. Preferably, the effective IL-1β concentration is 300 U/mL. In another preferred embodiment, the gp130 utilizing cytokine is IL-6. Preferably, the effective IL-6 concentration is 1000 U/mL of IL-6. In another preferred embodiment, the TNF superfamily member is TNFα. Preferably, the effective TNFα concentration is 1000 U/mL.

The most frequently used cocktail contains TNF-α, IL-1β and IL-6. Thus, in a preferred embodiment the pro-inflammatory cytokine cocktail comprises a mixture of It-1β, IL-6 and TNF-α. More preferably, the composition of the medium is 300 U/mL of IL-1β, 1000 U/mL of TNF-α and 1000 U/mL of IL-6.

In yet another embodiment, the pro-inflammatory cytokine cocktail further comprises a prostaglandin. More preferably, the prostaglandin utilized is prostaglandin E₂ (PGE₂). Preferably, the effective PGE₂ concentration is 1 µg/mL. More preferably, the composition of the medium is 300 U/mL of IL-1β, 1000 U/mL of TNF-α, 1000 U/mL of IL-6 and 1 µg/mL of PGE₂.

In another embodiment, the invention relates to a method for obtaining a antigen-loaded antigen-presenting cell *in vitro* comprising:
(i) placing the immature dendritic cell under conditions adequate for maturation of said immature dendritic cell into a mature dendritic cell and
(ii) contacting the mature dendritic cell with an immunogen comprising said antigen under conditions adequate for processing of the immunogen and presentation by the antigen-presenting cell.

Steps (i) and (ii) can be carried out sequentially or simultaneously. In a preferred embodiment, steps (i) and (ii) are carried out sequentially, i.e. the immature dendritic cells are first matured and then the mature dendritic cells are contacted with the antigen.

Step (i) is carried out wherein the conditions adequate for maturation of the immature dendritic cell into a mature dendritic cell comprise the incubation of said cell in a medium comprising a dendritic cell maturation agent selected from the group consisting of:
(i) a TLR4 ligand and
(ii) a pro-inflammatory cytokine cocktail.

In a preferred embodiment, the TLR4 ligand for use in the method according to the present invention include LPS. In another embodiment, the the pro-inflammatory cytokine cocktail comprises at least an agonist of the IL-1 receptor, a gp130 utilizing cytokine and a TNF superfamily member. Said cytokine cocktail can include other compounds. In a still more preferred embodiment, the IL-1 receptor agonist is IL-1β. Preferably, the effective IL-1β concentration is 300 U/mL. In another preferred embodiment, the gp130 utilizing cytokine is IL-6. Preferably, the effective IL-6 concentration is 1000 U/mL of IL-6. In another preferred embodiment, the TNF superfamily member is TNFα. Preferably, the effective TNFα concentration is 1000 U/mL.

The most frequently used cocktail contains TNF-α, IL-1β and IL-6. Thus, in a preferred embodiment the pro-inflammatory cytokine cocktail comprises a mixture of IL-1β, IL-6 and TNF-α. More preferably, the composition of the medium is 300 U/mL of IL-1β, 1000 U/mL of TNF-α and 1000 U/mL of IL-6.

In yet another embodiment, the pro-inflammatory cytokine cocktail further comprises a prostaglandin. More preferably, the prostaglandin utilized is prostaglandin E₂ (PGE₂). Preferably, the effective PGE₂ concentration is 1 µg/mL. More preferably, the composition of the medium is 300 U/mL of IL-1β, 1000 U/mL of TNF-α, 1000 U/mL of IL-6 and 1 µg/mL of PGE₂.

The second method of the invention allows obtaining dendritic cells loaded with HIV-1 viral particles that can be used as a dendritic cell vaccine in the same subject from which the HIV-1 viral particle was isolated. This has the advantage that the HIV-1 viral particle lacking a functional integrase protein is exactly the same group and subtype that the viral particle which infected the subject.

Furthermore, the dendritic cell used in the method of the invention may also be from the same subject. In a preferred embodiment, the dendritic cell is autologous to the subject from which the HIV-1 viral particle has been isolated.

The loading of a dendritic cell with the HIV-1 viral particle of the invention may be made using a mature dendritic cell or an immature dendritic cell. In a preferred embodiment of the second method of the invention, the loading of the dendritic cell is made with an immature dendritic cell. In another embodiment, the loading of the dendritic cell is made with a mature dendritic cell.

In preferred embodiments of the second method of the invention, the HIV-1 viral particle lacking a functional integrase protein and the method of loading a dendritic cell with said HIV-1 viral particle may be any HIV-1 viral particle and any method included amongst those described within the context of the first method of the invention.

In a preferred embodiment, the HIV-1 viral particle contains a full complement of viral proteins except integrase. In another preferred embodiment, the HIV-1 viral particle lacking a functional integrase protein contains a deletion in the integrase protein or lacks the integrase protein.

In another preferred embodiment, the HIV-1 is CXCR4-tropic.

The methods of the invention allow obtaining antigen-loaded dendritic cells suitable for their use as a vaccine.

Thus, another aspect of the present invention is an antigen-loaded antigen-presenting cell obtainable by the first method of the invention. Said antigen-loaded antigen-presenting cell has a higher ability to present antigens than cells obtained by other known methods.

Another aspect of the invention is an antigen-loaded antigen-presenting cell obtainable by the first method of the invention for use in medicine. In another aspect, the invention relates to an antigen-presenting cell obtainable by the first method of the invention for use in the treatment or prevention of a disease which requires an immune response against the antigen which is loaded in the antigen-presenting cell. In another aspect, the invention relates to the use of an antigen-presenting cell obtainable by the first method of the invention for the manufacture of a medicament for the treatment or prevention of a disease which requires an immune response against the antigen which is loaded in the antigen-presenting cell. In another aspect, the invention relates to a method for the treatment or prevention in a subject of a disease which requires an immune response against the antigen which is loaded in the antigen-presenting cell that comprises the administration to said subject of an antigen-presenting cell according to the invention.

In a preferred embodiment, the immunogen is a HIV immunogen and the disease is a HIV infection or a disease associated with a HIV infection. In a more preferred embodiment, the subject to be treated is the same subject from which the HIV-1 viral particle was isolated. In another preferred embodiment, the subject to which the immunogenic composition or vaccine is administered is under antiretroviral therapy.

The terms "medicament", "treatment", "prevention", "HIV infection" and "disease associated with a HIV infection" are defined in the context of the invention's immunogenic compositions and therapeutic uses thereof.

A further aspect of the present invention is a dendritic cell loaded with a HIV-1 viral particle lacking a functional integrase protein obtainable by the second method of the invention.

Conventional assays utilized to detect T cell responses include, for instance, proliferation assays, lymphokine secretion assays, direct cytotoxicity assays and limiting dilution assays. For example, to measure cellular immunity, cell suspensions of enriched CD4+ and CD8+ T cells from lymphoid tissues are used to quantify antigen-specific T cell responses by cytokine-specific ELISPOT assay. *See* Wu S, *et al.,* 1995, 1997, *supra.* Such assays can measure the numbers of antigen-specific T cells that secrete IL-2, IL-4, IL-5, IL-6, IL-10 and IFN-gamma. All ELISPOT assays are conducted using commercially-available capture and detection mAbs (R&D Systems, Inc., Minneapolis, MN, US; BD Biosciences Pharmingen, San Diego, CA, US). *See* Wu S, *et al.,* 1995, 1997, *supra*; Shata M, 2001, supra.

Assays to test the capacity of the antigen-loaded dendritic cells to present antigens are described in the general procedures of the experimental part of the present invention.

### 3. HIV-I immunogens lacking a functional integrase protein

The present invention discloses HIV-1 immunogens lacking a functional integrase protein which are capable of eliciting an immune response without replicating into the host cells. As shown in examples 1 to 3 of the present invention, a HIV-1 viral particle lacking a functional integrase protein is capable of being captured by a dendritic cell and activating CD4⁺ and CD8⁺ T cells without leading to any trans-infection of CD4+ cells.

Thus, an aspect of the present invention is a HIV-1 viral particle lacking a functional integrase protein. In a preferred embodiment, the HIV-1 viral particle lacks at least 95%, at least 96%, at least 97%, at least 98%, at least 99%, preferably, 100% of its function.

In a preferred embodiment, the HIV-1 viral particle contains a full complement of all the viral genes except the *pol* gene. In a more preferred embodiment, the HIV-1 viral particle lacking a functional integrase protein contains a deletion in the integrase protein or lacks the integrase protein completely. Preferably, the HIV-1 viral particle lacks the whole integrase protein.

In another preferred embodiment, the HIV-1 is CXCR4-tropic.

Said embodiments have been defined previously in the context of the first method of the invention.

### 4. Genomes, vectors and cells of the invention

The present invention also provides genomes encoding HIV-1 immunogens lacking a functional integrase protein and vectors and cells comprising them.

Thus, in another aspect, the invention relates to a HIV-1 viral genome characterized by lacking partially or completely the integrase encoding region.

The HIV-1 viral genome of the invention contains all genes of the HIV-1 viral genome except part of the integrase encoding region or the complete integrase encoding region.

The expression "the integrase encoding region", as used herein, refers to the region of the HIV-1 viral genome that encodes the integrase protein.

By "lacking partially or completely the integrase encoding region" it is understood that the integrase encoding region is not complete, either because part of the nucleotides have been deleted or because the whole encoding region has been deleted.

The HIV-1 viral genome of the invention may contain a deletion or mutation of other regions of the genome.

In a preferred embodiment, the partial or complete lack of the integrase encoding region is the single difference between said HIV-1 viral genome lacking partially or completely the integrase encoding region and a wild-type HIV-1 viral genome. In the present context, the term "wild-type HIV-1 viral genome" refers to a HIV-1 viral genome that is the typical genome of HIV-1 as it occurs in nature. The embodiment in which "the partial or complete lack of the integrase encoding region is the single difference between said HIV-1 viral genome lacking partially or completely the integrase encoding region and a wild-type HIV-1 viral genome" means that the HIV-1 viral genome of the invention lacking partially or completely the integrase encoding region is a HIV-1 viral genome identical to a wild-type HIV-1 viral genome except in that the integrase encoding region is partially or completely deleted.

In a more preferred embodiment, the whole integrase encoding region has been deleted. This means that the integrase protein is not synthesized. In an even more preferred embodiment, the nucleotides 4159 to 5124 of the *gag-pol* gene have been deleted.

In HIV-1, the gene encoding the viral protease, *pro,* and the gene expressing the polymerase and integrase, *pol,* are translated as part of a single Gag-Pro-Pol polyprotein precursor of 160 kDa (p160). Translation of Pro-Pol requires a -1 frameshift that allows reading through the *gag* stop codon. As a result of viral protease processing, the protease itself (PR), the reverse transcriptase heterodimer (RT, composed of a 66- and a 51-kDa subunit) and the integrase (IN) are released from the precursor. *See* Cimarelli A, et al., Cell. Mol. Life Sci. 2002; 59:1166-1184.

In another aspect, the invention relates to a vector comprising a genome as defined previously.

Thus, suitable vectors according to the present invention include prokaryotic vectors, such as pUC18, pUC19, and Bluescript plasmids and derivatives thereof, like the mp18, mp19, pBR322, pMB9, ColE1, pCR1 and RP4 plasmids; phages and shuttle vectors, such as pSA3 and pAT28 vectors; expression vectors in yeasts, such as 2-micron plasmid type vectors; integration plasmids; YEP vectors; centromeric plasmids and analogues; expression vectors in insect cells, such as the vectors of the pAC series and of the pVL series; expression vectors in plants, such as vectors of the pIBI, pEarleyGate, pAVA, pCAMBIA, pGSA, pGWB, pMDC, pMY, pORE series and analogues; and expression vectors in superior eukaryotic cells either based on viral vectors (e.g. adenoviruses, viruses associated to adenoviruses, retroviruses and lentiviruses) as well as non-viral vectors, such as the psilencer 4.1-CMV (Ambion, Life Technologies Corp., Carslbad, CA, US), pcDNA3, pcDNA3.1/hyg pHCMV/Zeo, pCR3.1, pEFI/His, pIND/GS, pRc/HCMV2, pSV40/Zeo2, pTRACER-HCMV, pUB6/VS-His, pVAXI, pZeoSV2, pCI, pSVL and pKSV-10, pBPV-1, pML2d and pTDT1 vectors.

The pUC-based pNL4-3 plasmid (14,833 bp in size) is the most widely used vector for *in vitro* manipulations of the HIV-1 proviral sequences. The HIV-1 vector pNL4-3 encodes all known HIV-1 gene products and its complete sequence is defined under accession number AF324493.2 in the GenBank database (release May 24^{th}, 2010). Thus, in a preferred embodiment the vector is pNL4-3 wherein the integrase region is partially or completely missing, preferably wherein the integrase encoding region is completely missing.

The present invention relates to a plasmid from which the integrase encoding region has been deleted by removing nucleotides 4160-5121 of the pNL4-3 vector. The resulting plasmid is named pNL4-3AIN having a sequence SEQ ID NO: 1. Thus, in a preferred embodiment the vector is the pNL4-3 vector wherein nucleotides 4160-5121 have been deleted. Preferably, the plasmid is pNL4-3AIN.

The plasmid pNL4-3AIN has been deposited prior to filing the present patent application in the Deutsche Sammlung von Mikroorganismen und Zellkulturen GmbH (DSMZ), Inhoffenstraße 7 B, D-38124 Braunschweig, Germany, as legally recognized institution for that purpose in accordance with the Budapest Treaty, of April 28^{th}, 1977, on the International Recognition of the Deposit of Microorganisms. The DSMZ has assigned to the plasmid pNL4-3ΔIN the deposit number DSM 23817. Thus, in a more preferred embodiment the vector is the plasmid DSM 23817.

In another aspect, the invention relates to a cell comprising a HIV-1 viral genome according to the invention or a vector according to the invention.

Cells suitable for the expression of the immunogens of the invention include, without limitation, mammal, plant, insect, fungal and bacterial cells. Bacterial cells include, without being limited to, Gram-positive bacterial cells such as species of the *Bacillus, Streptomyces* and *Staphylococcus* genus and Gram-negative bacterial cells such as cells of the *Escherichia* and *Pseudomonas* genus. Fungal cells include, preferably, cells of yeasts such as *Saccharomyces*, *Pichia pastoris* and *Hansenula polymorpha.* Insect cells include, without being limited to, *Drosophila* cells and Sf9 cells. Plant cells include, among others, cells of crop plants such as cereals, medicinal, ornamental or bulbous plants. Mammalian cells include, without limitation, Chinese hamster ovary (CHO) cells such as CHO-K1 (ATCC accession number CCL-61), DG44 (Chasin L, et al., Som. Cell Mol. Genet. 1986; 12:555-556 and Kolkekar A, et al., , Biochemistry 1997; 36:10901-10909), CHO-K1 Tet-On (Clontech Inc., Mountain View, CA, US), CHO designated ECACC 85050302 (CAMR, Salisbury, Wiltshire, UK), CHO clone 13 (GEIMG, Genova, IT), CHO clone B (GEIMG, Genova, IT), CH0-K1/SF designated ECACC 93061607 (CAMR, Salisbury, Wiltshire, UK), RR-CHOKI designated ECACC 92052129 (CAMR, Salisbury, Wiltshire, UK), CHO cells negative for dihydrofolate reductase (CHO/-DHFR, Urlaub G, Chasin L, Proc. Natl. Acad. Sci. USA; 77:4216-4220), SV40-transformed monkey kidney CV1 cells (COS, COS-7, ATCC accession number CRL-1651); human embryonic kidney cells (293, 293T cells); baby hamster kidney cells (BHK, ATCC accession number CCL-IO); monkey kidney cells (CVI, ATCC accession number CCL-70); African Green Monkey kidney cells (VERO-76, ATCC accession number CRL-1587; VERO, ATCC accession number CCL-81); mouse Sertoli cells (TM4, Mather J, Biol. Reprod. 1980; 23:243-251); human cervical carcinoma cells (HELA, ATCC accession number CCL-2); dog kidney cells (MDCK, ATCC accession number CCL-34); human lung cells (W138, ATCC accession number CCL-75); human hepatoma cells (HEP-G2, HB 8065); mouse mammary tumor cells (MMT 060562, ATCC accession number CCL-51); buffalo rat liver cells (BRL 3A, ATCC accession number CRL- 1442); TRI cells (Mather J, Ann. NY Acad. Sci. 1982, 383:44-68); MCR 5 cells and FS4 cells. Preferably, the host cells are HEK-293T cells. In another preferred embodiment, said cell is a dendritic cell.

### 5. Immunogenic compositions and therapeutic uses thereof

The HIV-1 viral particles, HIV-1 viral genomes, vectors or cells of the invention can also be used to prepare an immunogenic composition. Thus, another aspect of the present invention is an immunogenic composition or a vaccine comprising a HIV-1 viral particle, a HIV-1 viral genome, a vector or a cell according to the invention.

The terms "HIV-1 viral particle", "HIV-1 viral genome", "vector" and "cell" have been defined in the context of the previous aspects of the invention. The term "cell" refers to cells obtainable by the first method of the invention and also to cells obtainable by the second method of the invention.

In a further aspect, the invention relates to a HIV-1 viral genome, a vector, a cell or an immunogenic composition or vaccine according to the invention for use in medicine. The immunogenic composition or vaccine of the invention contains at least a pharmaceutically acceptable carrier.

The carrier can contain additional agents such as wetting or emulsifying agents, pH buffering agents, or adjuvants that enhance the effectiveness of the formulation. Usable adjuvants may enhance the immunological response by activating antigen presenting cells (APC), macrophages or stimulating specific sets of lymphocites.

An adjuvant according to the present invention may be any ligand suitable for the activation of a pathogen recognition receptor (PRR) expressed in and on dendritic cells (DCs), T cells, B-cells or other antigen presenting cells. Ligands activating the nucleotide-binding oligomerization domain (NOD) receptor pathway may be suited for the purpose of the invention. Adjuvants suitable for these ligands may be muramyl dipeptide derivatives. Ligands activating the toll-like receptors (TLRs) may also convene for the purpose of the invention. Those receptors are member of the PRR family and are widely expressed on a variety of innate immune cells, including DCs, macrophages, mast cells and neutrophils.

Adjuvants could also be selected, for example, from the group including AlK(SO₄)₂, AlNa(SO₄)₂, AlNH₄(SO₄), silica, alum, Al(OH)₃, Ca₃(PO₄)₂, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyl-L-alanyl-D-isoglutaminyl-L-alanine-2-(1'2'-dipalmitoyl-sn-glycero-3-hydroxphosphoryloxy)-ethylamine (CGP 19835A, also referred to as MTP- PE), RIBI (MPL+TDM+CWS) in a 2 percent squalene/TWEEN® 80 emulsion, lipopolysaccharides and its various derivatives, including lipid A, Freund's Complete Adjuvant (FCA), Freund's Incomplete Adjuvants, Merck Adjuvant 65, polynucleotides (e.g. poly IC and poly AU acids), wax D from *Mycobacterium tuberculosis,* substances found in *Corynebacterium parvum, Bordetella pertussis*, and members of the genus *Brucella,* Titermax, ISCOMS, Quil A, ALUN, Lipid A derivatives, choleratoxin derivatives, HSP derivatives, LPS derivatives, MPL derivatives, synthetic peptide matrixes or GMDP, interleukin 1, interleukin 2, Montanide ISA-51 and QS-21, CpG oligonucleotide, poly I:C, and IL-12, IL-15 or GM-CSF. *See* Hunter R, US 5,554,372 and Jager E, Knuth A, WO1997028816

For pharmaceutical compositions comprising an agent that is a nucleic acid molecule, the nucleic acid molecule may be present within a number of delivery systems known in the art, including nucleic acid, bacterial, viral and mammalian expression systems such as, for example, recombinant expression constructs as provided herein. Techniques for incorporating DNA into such expression systems have been disclosed previously. *See* Ulmer J, et al., Science 1993; 259:1745-1749 and Cohen J, Science 1993; 259:1691-1692. The DNA may also be "naked". The uptake of naked DNA may be increased by coating the DNA onto biodegradable beads, which are efficiently transported into the cells.

Nucleic acid molecules may be delivered to cells by any of several methods known in the art. *See* Akhtar S, et al., Trends Cell Biol. 1992; 2:139-144, Maurer N, et al., Mol. Membr. Biol. 1999; 16:129-140, Selbo P, et al., Int. J. Cancer 2000; 87:853-859, Selbo P, et al., Tumour Biol. 2002; 23:103-112, Sullivan S, et al., WO1994002595, Hoffman A, et al., US 20010007666, MacLachlan I, US 20030077829, and Beigelman L, et al., US 6,395,713. These delivery methods include, but are not limited to, encapsulation in liposomes, by iontophoresis, or by incorporation into other vehicles (e.g. biodegradable polymers, hydrogels, cyclodextrins, poly(lactic-co-glycolic)acid (PLGA) and PLCA microspheres, biodegradable nanocapsules, bioadhesive microspheres, proteinaceous vectors). *See* González H, et al., Bioconjug. Chem. 1999; 10:1068-1074, Castor T, US 20020130430, O'Hare P, et al., WO 2000053722, Wang L, WO2003046185, Wang L, et al., WO2003047518, and Vook N, et al., US 6,447,796. In another embodiment, the nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives. *See* MacLachlan I, US 20030077829.

In a further aspect, the invention relates to a HIV-1 viral genome, a vector, a cell or an immunogenic composition or vaccine according to the invention for use in the treatment or prevention of a HIV infection or a disease associated with a HIV infection. Alternatively, the invention relates to the use of a HIV-1 viral genome, a vector, a cell or an immunogenic composition or a vaccine according to the invention for the manufacture of a medicament for the treatment or prevention of a HIV infection or a disease associated with a HIV infection. Alternatively, the invention relates to a method for the treatment or prevention in a subject of a HIV infection or a disease associated with a HIV infection that comprises the administration to said subject of a HIV-1 viral genome, a vector, a cell or an immunogenic composition or a vaccine according to the invention.

The beneficial treatment or preventive effects of a HIV immunogenic composition in relation to HIV infection or AIDS symptoms include, for example, preventing or delaying initial infection of an individual exposed to HIV; reducing viral burden in an individual infected with HIV; prolonging the asymptomatic phase of HIV infection; maintaining low viral loads in HIV infected patients whose virus levels have been lowered via anti-retroviral therapy (ART); increasing levels of CD4 T cells or lessening the decrease in CD4 T cells, both HIV-1 specific and non-specific, in drug naive patients and in patients treated with ART, increasing overall health or quality of life in an individual with AIDS; and prolonging life expectancy of an individual with AIDS. A clinician can compare the effect of immunization with the patient's condition prior to treatment, or with the expected condition of an untreated patient, to determine whether the treatment is effective in inhibiting AIDS. In a preferred embodiment, the immunogenic compositions of the invention are preventive compositions.

The immunogenic compositions of the invention may be useful in the treatment of a HIV-1 infection. While all animals that can be afflicted with HIV-1 or their equivalents can be treated in this manner (e.g. chimpanzees, macaques, baboons or humans), the immunogenic compositions of the invention are directed particularly to their therapeutic uses in humans. Often, more than one administration may be required to bring about the desired therapeutic effect; the exact protocol (dosage and frequency) can be established by standard clinical procedures.

The present invention further relates to preventing or reducing symptoms associated with HIV infection. These include symptoms associated with the minor symptomatic phase of HIV infection, including, for example, shingles, skin rash and nail infections, mouth sores, recurrent nose and throat infection and weight loss. In addition, further symptoms associated with the major symptomatic phase of HIV infection, include, for instance, oral and vaginal thrush (*Candida*), persistent diarrhea, weight loss, persistent cough and reactivated tuberculosis or recurrent herpes infections, such as cold sores (*herpes simplex*). Other symptoms of full-blown AIDS which can be treated in accordance with the present invention include, for instance, diarrhoea, nausea and vomiting, thrush and mouth sores, persistent, recurrent vaginal infections and cervical cancer, persistent generalized lymphadenopathy (PGL), severe skin infections, warts and ringworm, respiratory infections, pneumonia, especially *Pneumocystis carinii* pneumonia (PCP), *herpes zoster* (or shingles), nervous system problems, such as pains, numbness or "pins and needles" in the hands and feet, neurological abnormalities, Kaposi's sarcoma, lymphoma, tuberculosis or other similar opportunistic infections.

In a preferred embodiment, the immunogenic compositions or vaccines according to the invention are administered to the subject from whom the viral particle or dendritic cells were isolated. Thus, in a preferred embodiment the subject to be treated is the same subject from which the HIV-1 viral particle was isolated.

In another preferred embodiment, the subject to which the immunogenic composition or vaccine is administered is under antiretroviral therapy (ART), preferably under highly active antiretroviral therapy (HAART).

All publications mentioned hereinabove are hereby incorporated in their entirety by reference.

While the foregoing invention has been described in some detail for purposes of clarity and understanding, it will be appreciated by one skilled in the art from a reading of this disclosure that various changes in form and detail can be made without departing from the true scope of the invention and appended claims.

### A. General Procedures

### 1. Cellular cultures

Peripheral blood cells from HIV-1-seronegative donors were provided by the Banc de Sang i de Teixits (BST, University Hospital Germans Trias i Pujol, Badalona, ES) or purchased (EFS, Etablissement Français du Sang, Pitié-Salpêtrière Hospital, Paris, FR). Peripheral blood mononuclear cells (PBMCs) were obtained from a standard Ficoll density gradient centrifugation. Purified monocyte populations were isolated with CD14⁺ selection magnetic beads (Miltenyi Biotec GmbH, Bergisch Gladbach, DE) and cultured with RPMI 1640 containing 10% fetal bovine serum (Invitrogen Corp., Carslbad, CA, US), 1000 U/ml of GM-CSF and IL-4 (R&D) for 5 days. Culture medium containing GM-CSF and IL-4 was replaced on days 2 and 5 of dendritic cell (DC) differentiation. On day 5 DCs were matured by adding: i) 100 ng/ml of LPS (Sigma-Aldrich Co., Saint Louis, MO, US) or ii) 300 U/ml IL-1β, 1000 U/ml IL-6, 1000 U/ml TNF-α (CellGenix GmbH, Freiburg, DE) and 1µg/ml PGE₂ (Sigma-Aldrich Co., Saint Louis, MO, US) (hereinafter referred to as "ITIP"). Monocytes after isolation from PBMCs, immature monocyte-derived DCs (iDCs) and fully mature monocyte-derived DC (mDCs) at day 7 were immunophenotyped by flow cytometry (FACSCalibur Flow Cytometer; BD Biosciences Corp., Franklin Lakes, NJ, US). The monoclonal antibodies (mAbs) used for cell immunophenotyping were: CD14-FITC and -PE (clone M5E2, Pharmingen, BD Biosciences Corp., Franklin Lakes, NJ, US), CD209-PE and -APC (clone DCN46, Pharmingen, BD Biosciences Corp., Franklin Lakes, NJ, US), CD4-PerCP (clone SK3, BD Biosciences Corp., Franklin Lakes, NJ, US), HLA-DR-PE and -PerCP (clone L243, BD Biosciences Corp., Franklin Lakes, NJ, US), HLA-ClassI-FITC (clone W6/32, (Sigma-Aldrich Co., Saint Louis, MO, US), CD86-FITC (clone 2331, Pharmingen, BD Biosciences Corp., Franklin Lakes, NJ, US), CD83-PE and -APC (clone HB15e, Pharmingen, BD Biosciences Corp., Franklin Lakes, NJ, US), and CD80-PE-Cy5 (clone L307.4, Pharmingen, BD Biosciences Corp., Franklin Lakes, NJ, US).

TZM-bl (NIH AIDS Research and Reference Reagent Program, NIH, Bethesda, MD, US and Tranzyme, Inc., Durham, NC, US) and HEK-293T cell lines were maintained in Dulbecco's modified Eagle's medium (DMEM) (Invitrogen Corp., Carslbad, CA, US) supplemented with 10% fetal bovine serum. TZM-bl is a genetically engineered HeLa cell line that expresses CD4, CXCR4 and CCR5 and contains Tatinducible luciferase and β-Gal reporter genes. *See* Wei X, et al., Antimicrob. Agents Chemother. 2002; 46(6):1896-1905. All media contained 100 U/ml of penicillin and 100 µg/ml of streptomycin (Invitrogen Corp., Carslbad, CA, US).

### 2. HIV-specific T cell clones

The N2 and F12 CD4⁺ T cell clones specific for HIV p24^{gag} (aa 271-290) and restricted by HLA-DRβ*04 or HLA-DRβ*01 respectively were used to monitor HLA-II antigen presentation. EM40-F21 and SL9-3 CD8⁺ T cell clones specific for HIV p17^{gag} (aa 77-85, SL9 peptide) and restricted by HLA-A2 were used to monitor HLA-I antigen presentation. The T cell clones were re-stimulated and expanded as previously described using irradiated feeders and autologous lymphoblastoid cell lines (LCL) loaded with cognate peptides in T cell cloning medium (RPMI 1640 containing 5% serum AB (SAB), 100 U/ml rhIL-2 and 1 µg/ml phytohemagglutinin (PHA) (PAA) complemented with non-essential amino-acid and sodium pyruvate (Gibco, Invitrogen Corp., Carslbad, CA, US). At least 4h prior to co-cultures with DC, T cell clones were thawed and allowed to rest in cloning medium without PHA. *See* Moris A, et al., Blood 2004; 103(7):2648-2654 and Moris A, et al., Blood 2006; 108(5):1643-1651.

### 3. Viral stocks and plasmids

Replication-competent full-length CXCR4-tropic HIV-1 (HIV_{NL4-3}) and integrase-deficient CXCR4-tropic HIV-1 (HIV_{NL4-3ΔIN}) stocks were generated transfecting the proviral construct pNL4-3 (NIH AIDS Research and Reference Reagent Program, NIH, Bethesda, MD, US) and pNL4-3ΔIN with calcium phosphate (CalPhos; BD Biosciences Corp., Franklin Lakes, NJ, US) to HEK-293T cells. The proviral vector pNL4-3 was engineered for generating the integrase-deficient CXCR4-tropic HIV-1 vector pNL4-3ΔIN (SEQ ID NO: 1). Both pNL4-3 and pNL4-3ΔIN were modified to express the described optimal (SLYNTVATL) (SEQ ID NO: 2) and escape (SLFNTIAVL) (SEQ ID NO: 3) epitope for p17^{gag} 77-85 (SL9 epitope) restricted by HLA-A2. *See* Llano A, et al., How to optimally define optimal cytotoxic t-lymphocyte epitopes in HIV infection? in Korber B, et al., Eds., "Theoretical Biology and Biophysics" (Los Alamos National Laboratory, Los Alamos, New Mexico, US, 2009) and Iversen A, et al., Nat. Immunol. 2006; 7:179-189. Supernatants containing virus were collected 48 h after transfection, later filtered (Millex HV, 0.45 µm; Millipore Corp., Billerica, MA, US), concentrated using the Lenti-X Concentrator kit (Clontech Labs., Inc., Mountain View, CA, US) and frozen at -80°C until use.

For the viral fusion assay, HIV-1 virions containing the β-lactamase-Vpr (BlaM-Vpr) chimera were produced as previously described. *See* Cavrois M, et al., Nat. Biotechnol. 2002; 20:1151-1154. In brief, HEK-293T cells were co-transfected with 60 µg of pNL4-3 or pNL4-3ΔIN proviral DNA, 20 µg of pCMV-BlaM-Vpr (Addgene Inc., Cambridge, MA, US), and 10 µg of pAdVAntage vectors (Promega Corp., Madison, WI, US). Supernatants containing virus were collected 48 h later, filtered (Millex HV, 0.45 µm; Millipore Corp., Billerica, MA, US), ultracentrifuged at 72,000 g for 90 min at 4°C and frozen at -80°C until use. The p24^{Gag} content of all viral stocks was determined by an ELISA assay (PerkinElmer Inc., Waltham, MA, US). Titers of all viruses were determined by using TZM-bl indicator cell line. *See* Li M, et al., J. Virol. 2005; 79(16): 10108-10125. Cells were assayed for luciferase activity 48 h after the infection (Bright-Glo Luciferase Assay System, Promega Corp., Madison, WI, US) in a Fluoroskan Ascent FL Luminometer (Thermo Fisher Scientific Inc., Waltham, MA, US).

### 4. Viral fusion assay

The viral fusion assay was performed as previously described. *See* Li, 2005, *supra.* Briefly, 5 x 10⁵ Jurkat T cells were infected with 400 ng p24^{Gag} of HIV_{NL4-3} or HIV_{NL4-3ΔIN} containing BlaM-Vpr for 4 h (spinoculation at 600 g for 90 min at 22°C and incubation for 2.5 h at 37°C 5% CO₂) in the presence or absence of 5 µg/ml C34 fusion inhibitor (NIH AIDS Research and Reference Reagent Program, NIH, Bethesda, MD, US). Cells were then washed in CO₂-independent medium (Gibco, Invitrogen Corp., Carslbad, CA, US) to remove free virions and loaded with 1mM CCF2-AM dye (Invitrogen Corp., Carslbad, CA, US) for 1 h at room temperature as recommended by the manufacturer. After two washes with development medium (CO₂-independent medium supplemented with 10% FBS), the BlaM cleavage of CCF2 reaction was allowed to proceed in dark for 16 h at room temperature in 200 µl of development medium. Finally, cells were washed in PBS and fixed in a 1.2% paraformaldehyde solution. The degradation of CCF2-AM by BlaM cleavage and its change in emission fluorescence was measured by flow cytometry using a BD LSR II (BD Biosciences Corp., Franklin Lakes, NJ, US). Data were collected with FACSDiva software (BD Biosciences Corp., Franklin Lakes, NJ, US) and analyzed with FlowJo software (Tree Star Inc., Ashland, OR, US).

### 5. Dendritic cell viral capture and trans-infection assays

A total of 2.5 x 10⁵ iDCs and mDCs matured for 48h with ITIP (mDC ITIP) or with LPS (mDC LPS) from at least 7 different donors were incubated at 37°C for 6 h with 50 ng p24^{Gag} of HIV_{NL4-3} or HIV_{NL4-3ΔIN} at a final concentration of 1 x 10⁶ cells/ml. During viral pulse, a portion of iDCs was matured with ITIP (iDC+ITIP) and another with LPS (iDC+LPS). After incubation, cells were extensively washed with phosphate-buffered saline (PBS) to remove uncaptured viral particles, and lysed with 0.5% Triton X-100 at final concentration of 5 x 10⁵ cells/ml. Lysates were cleared of cell debris by centrifugation to measure intracellular p24^{Gag} antigen content by an ELISA assay (PerkinElmer Inc., Waltham, MA, US) as previously described. *See* Izquierdo-Useros N, et al., J. Virol. 2007; 81:7559-7570. Before lysis, 10⁴ HIV-1-pulsed DCs were co-cultured with the reporter cell line TZM-bl at a ratio of 1:1 per well in a 96-well plate, at a final volume of 100 µl. Cells were assayed for luciferase activity after 48 h of co-culture (Brighty-Glo Luciferase Assay System, Promega Corp., Madison, WI, US) in a Fluoroskan Ascent FL Luminometer (Thermo Fisher Scientific Inc., Waltham, MA, US). Background values consisting of non-HIV-exposed DC-TZM-bl co-culture were subtracted for each cell condition.

### 6. Antigen presentation assay

For HLA-I antigen presentation experiments, PBMCs from HLA-A2⁺ donors were used to generate DCs. iDCs and mDCs matured for 48 h with ITIP (mDC ITIP) or with LPS (mDC LPS) were exposed for 24 h to the indicated viruses (500 ng p24^{Gag}/ml per 1 x 10⁶ cells) at 37°C and 5% CO₂ in culture medium with IL-4, GM-CSF, 5 µM azidothymidine (AZT) and 1.2 µM nevirapine (NVP) (Sigma-Aldrich Co., Saint Louis, MO, US). Alternatively, iDCs were loaded with HIV as before and simultaneously matured with ITIP (iDC+ITIP) or with LPS (iDC+LPS). Cells were then extensively washed with PBS to remove uncaptured viruses, and co-cultured for 16-18 h with EM40-F21 or SL9-2 CTL clones. T cell activation was monitored using IFNγ-ELISPOT assay as previously described. *See* Moris, 2004, *supra.* As positive control, DCs were loaded with 0.1 µg/ml of cognate peptide in the same conditions that HIV-pulsed cells with optimal activation and extensively washed prior co-culture with T cells. Negative control values consisting of non-HIV-exposed DC-CD8⁺ T cell clone co-culture were subtracted.

For HLA-II antigen presentation experiments, PBMCs from HLA-DRβ*04 or HLA-DRβ*01 donors were used to generate DCs. iDCs and mDCs matured for 48 h with ITIP (mDC ITIP) or with LPS (mDC LPS) were exposed for 6 h to the indicated viruses (285 ng p24^{Gag}/ml per 1 x 10⁶ cells) at 37°C and 5% CO₂ in culture medium with 5 µM AZT and 1.2 µM NVP (Sigma-Aldrich Co., Saint Louis, MO, US). Then, cells were washed extensively with PBS, to remove unbound viruses, and were cultured overnight in a medium with IL-4, GM-CSF, AZT and NVP. Alternatively, iDCs were loaded with HIV and simultaneously matured with ITIP (iDC+ITIP) or with LPS (iDC+LPS). Then, cells were washed with PBS and co-cultured for 16-18 h with N2 or F12 CD4⁺ T cell clones. T cell activation was monitored in an IFNγ-ELISPOT assay. As positive control, DCs were loaded with 0.1 µg/ml of cognate peptide in the same conditions that HIV-pulsed cells with optimal activation. Negative control values consisting of non-HIV-exposed DC-CD4⁺ T cell clone co-culture were subtracted.

### 7. Electron microscopy and statistical analysis

For electron microscopy analysis of viral capture by DCs, cells were processed as previously described. *See* Izquierdo-Useros, 2007, *supra.* In brief, 3 x 10⁶ mDC LPS were pulsed at 37°C overnight with 1,600 ng p24^{Gag} of HIV_{NL4-3} or HIV_{NL4-3A}IN. Then, cells were washed extensively with PBS and fixed in 2.5% glutaraldehyde for 1 h. Next, cells were processed for analysis of ultrathin sections using a Jeol JEM 1010 electron microscope (Jeol Ltd., Tokyo, JP). All statistical analyses were performed using GraphPad software Prism v.5 (GraphPad Software, Inc., La Jolla, CA, US).

### B. Examples

### Example 1

### DC maturation and HIV-1 capture

Fully mature DCs were obtained by culturing immature DCs (iDCs) with ITIP (mDC ITIP) or LPS (mDC LPS) for 48h. Then, iDCs and fully mature DCs were incubated with HIV_{NL4-3} or HIV_{NL4-3ΔIN} for 6 h. After extensive washing, part of the cells was lysed and DC-associated HIV-1 was determined. The remaining cells were co-cultured with the reporter cell line TZM-bl to assay the DC-mediated *trans*-infection. DC maturation with LPS (mDC LPS) enhanced HIV-1 capture compared with iDC (>5-fold, *P*=0.0039, Wilcoxon matched pairs test) and resulted in a higher *trans*-infection of HIV_{NL4-3} to target cells (>7-fold, *P*=0.0156). The HIV_{NL4-3AIN} was captured with the same efficiency than the wild-type HIV_{NL4-3} by DC (P=NS, Wilcoxon matched pairs test). *See* Fig. 1. Unexpectedly, DC maturation with ITIP (mDC ITIP) increased neither uptake nor transmission of HIV_{NL4-3} and remained at levels similar to iDCs. The phenotypic markers of maturation and differentiation between mDC LPS and mDC ITIP did not diverge. *See* Figs. 3 and 4.

Then, the viral uptake and *trans*-infection abilities of DCs matured with LPS (iDC+LPS) or with ITIP (iDC+ITIP) during viral capture were compared with DC fully matured prior HIV-1 incubation. The objective of this comparison was to evaluate if the maturation of DCs during antigen loading had any influence over viral capture and subsequent HIV-1 transmission to susceptible cells. *See* Ahlers J, et al., Trends Mol. Med. 2009; 15:263-274 and Palucka K, et al., Immunity 2010; 33:464-478. Surprisingly, not only iDC+ITIP, but also iDC+LPS, exhibited poor capture and transmission capacities relative to iDCs. *See* Fig. 1. DC maturation with LPS during viral pulse (iDC+LPS) did not enhance HIV-1 capture to levels observed in mDC LPS. Therefore, DC fully matured with LPS (mDC LPS) retained the greatest capacity to capture and transmit HIV-1 to susceptible cells. These results show that HIV-1 capture and transmission mediated by DC depend not only on the DC maturation state, but also on the activation stimuli used for maturation, as well as on the time lag between DC maturation and antigen loading. *See* Izquierdo-Useros, 2007, *supra* and Sanders R, et al., J. Virol. 2002; 76:7812-7821.

In all cell subsets, viral capture correlates with viral *trans*-infection of HIV_{NL4-3} to target cells, with higher ability of mDC LPS (P=0.0156, Wilcoxon matched pairs test). DC-mediated *trans*-infection of HIV_{NL4-3AIN} was completely abrogated in all cell conditions (P=0.0156, Wilcoxon matched pairs test).

On the other hand, HIV_{NL4-3AIN} was captured by DC with the same efficiency than the replicative-competent HIV_{NL4-3}. *See* Fig. 1. Then, the preservation of the HIV_{NL4-3ΔIN} envelope integrity and functionality was analyzed. Through viral fusion assays, HIV_{NL4-3AIN} was demonstrated to be as fusogenic as the wild type HIV_{NL4-3} and equally susceptible to the C34 fusion inhibitor, even though its whole integrase encoding region was missing. *See* Fig. 5A; Cavrois, 2002, *supra.*

Additionally, to evaluate whether the HIV_{NL4-3ΔIN} followed the same intracellular trafficking that the wild type HIV_{NL4-3} in DC, both viral particles were monitored in parallel by electron microscopy in mDC LPS. The HIV_{NL4-3} and HIV_{NL4-3ΔIN} virions displayed an identical structure, with a characteristic electron-dense core and similar accumulation in intracellular compartments. *See* Figs. 5A and 5B. These findings indicate that the lack of integrase in HIV_{NL4-3ΔIN} does not alter viral fusogenicity and morphology and that HIV_{NL4-3ΔIN} behaves as the wild type virus for all practical purposes, albeit not infectious.

### Example 2

### Viral capture and T cell activation

The abilities of iDCs, mDC ITIP, mDC LPS, iDC+ITIP and iDC+LPS to present HIV-1-derived antigens to CD4⁺ and CD8⁺ T cells was evaluated in order to elucidate, if any, the correlation between antigen presentation and T cell activation to viral capture. Several HIV-specific T cell clones previously generated were utilized for this purpose. To monitor HLA-I HIV-1 antigen presentation, two different CD8+ T cell clones EM40-F21 and SL9-2 specific for HIV-1 p17^{Gag} (aa 77-85) and restricted by HLA-A*02 were used. *See* Moris, 2004, *supra.* As the wild type HIV_{NL4-3} and its derived HIV_{NL4-3AIN} did not present the consensus SL9 epitope restricted by HLA-A*02, HIV_{NL4-3} and HIV_{NL4-3ΔIN} were engineered to express the optimal SL9 sequence (SLYNTVATL) (SEQ ID NO: 2) or the escape variant (SLFNTIAVL) (SEQ ID NO: 3) of SL9 epitope. *See* Llano, 2009, and Iversen, 2006, *supra.* Two CD4⁺ T cell clones N2 and F12 specific for HIV p24^{Gag} (aa 271-290) and restricted by HLA-DRβ*04 or HLA-DRβ*01, respectively, were used to evaluate HLA-II antigen presentation. *See* Moris, 2005, *supra.* HLA-matched DCs for each HIV-specific T cell clone were exposed for 24h (HLA-I assays) or 6h (HLA-II assays) to HIV_{NL4-3} or HIV_{NL4-3ΔIN.} T cell activation was monitored using IFN-γ ELISPOT after overnight co-culture of HIV-pulsed DCs with HIV-specific T cell clones. *See* Fig. 2. All assays were performed in the presence of NVP and AZT to prevent viral replication and guarantee that activation of HIV-specific CD8⁺ and CD4⁺ T cell clones was not due to the presentation of *de novo* viral synthesis in DCs.

HIV_{NL4-3} or HIV_{NL4-3ΔIN} loaded mDC LPS did not enhance HIV-specific CD8⁺ T cell activation as compared to iDCs. *See* Fig. 2. This was in contrast to the extremely high viral capture and HLA-molecule expression observed with mDC LPS. *See* Figs. 1, 3A and 3C. Thus, increased viral capture does not correlate with better T cell activation.

Inducing full DC maturation with ITIP had a moderate effect, if any, on HIV-specific CTL activation. *See* Fig. 2. It has been shown previously that HLA-I restricted exogenous HIV antigen presentation requires fusion of viral and cellular membranes in a CD4/coreceptor-dependent manner and release of HIV-1^{Gag} capsid into the cytosol of DCs for proteasomal processing and HLA-I loading. *See* Buseyne F, et al., Nat. Med. 2001; 7:344-349 and Sabado R, et al., Eur. J. Immunol. 2007; 37:1752-1763. However, DC maturation is associated with a decline in HIV-1 fusion, which, in turn, has a direct impact in the ability of mature DCs to support viral replication. *See* Cavrois, 2002, *supra,* and Dong C, et al., J. Virol. 2007; 81:11352-11362. This may explain why DCs matured with either LPS or ITIP exhibiting high levels of HLA-I and co-stimulatory molecules induced a low stimulation of HIV-specific CD8⁺ T cell clones.

In spite of their high viral capture ability, mDC LPS did not enhanced the HIV-specific CD4⁺ activation loaded with HIV_{NL4-3} or HIV_{NL4-3ΔIN} (stated in Figure 1A).

Interestingly, as compared to mDC ITIP or mDC LPS, iDCs induced a 3-fold enhancement of HIV-specific CD4⁺ T cell clone activation, that is in contrast to their reduced expression of HLA-II and co-stimulatory molecules and their poor capacity to capture HIV-1 virions. *See* Figs. 1, 2, 3B and 3D. These results strongly suggest that HIV-1 capture by mDC LPS does not route HIV virions towards degradation compartments and HLA loading.

### Example 3

### DC maturation with LPS during viral capture and HLA-I and HLA II antigen presentation

The effect of maturation of iDC during HIV-1 loading in antigen presentation by HLA-I and HLA-II molecules was next examined. For HLA-I antigen presentation assays, iDCs were matured with ITIP or LPS (iDC+ITIP and iDC+LPS, respectively) and simultaneously pulsed with HIV_{NL4-3} or HIV_{NL4-3ΔIN} expressing the optimal (SLYNTVATL) (SEQ ID NO: 2) or the escape variant (SLFNTIAVL) (SEQ ID NO: 3) of SL9 epitope for 24h. *See* Llano, 2009, and Iversen, 2006, *supra.* For HLA-II experiments, iDCs were incubated with HIV_{NL4-3} or HIV_{NL4-3ΔIN} for 6h and then matured with ITIP or LPS (iDC+ITIP and iDC+LPS, respectively). After co-culturing overnight HIV-pulsed DCs with HIV-specific T cell clones, antigen presentation was quantified by IFN-γ ELISPOT. *See* Fig. 2. All assays were performed in the presence of NVP and AZT to ensure that antigens do not derived from neo-synthesized HIV proteins.

The EM40-F21 HIV p17^{Gag} SL9 CD8⁺ T cell clone (aa 77-85) restricted by HLA-A2 was used for the analysis of exogenous HLA-I antigen presentation. The N2 HIV p24^{Gag} CD4⁺ T cell clone (aa 271-290) restricted by HLA-DRβ*04 was utilized for evaluating the HLA-II antigen presentation.

In contrast to mDC ITIP and mDC LPS, HIV-loaded iDC+ITIP and iDC+LPS induced a higher activation of HIV-specific CD8⁺ T cells than iDCs. *See* Fig. 2. Both HIV_{NL4-3} and HIV_{NL4-3ΔIN} were equally cross-presented to the HIV-specific CD8⁺ T cells. Minor differences were observed in HLA-I antigen presentation between LPS and ITIP maturing DC, though iDC+LPS represented a >3-fold increase in IFN-γ secretion and ITIP a >1.5-fold change comparing to their fully matured counterparts (mDC LPS and mDC ITIP, respectively). It is worth noting that neither enhanced viral capture nor HLA-I molecule expression correlated with better CD8⁺ T cell activation. See Fig. 3A and 3C. As expected, both HIV_{NL4-3} and HIV_{NL4-3ΔIN} expressing the escape variant (SLFNTIAVL) (SEQ ID NO: 3) of SL9 epitope did not induce responses in the EM40-F21 CD8⁺ T cell clone in any DC condition. See Fig. 6. As previously stated, DC maturation restricts fusion of viral and cell membranes, but HIV fusion is crucial for cytosolic proteasomal processing of Gag proteins and proper HLA-I antigen presentation. *See* Buseyne, 2001, Cavrois, 2002 and Sabado, 2007, *supra.* Although iDCs capture lower amounts of virions, HIV-1 is more able to fuse. This property might be helpful to enhance antigen uptake and the HLA-I antigen presentation of HIV-derived peptides.

As compared to mDC LPS and mDC ITIP, LPS maturation of iDCs after viral pulse (iDC+LPS) substantially increased the activation of the HIV-specific CD4⁺ T cells. *See* Fig. 2. Once more, the capacity to activate HIV-specific CD4+ T cell clones did not correlate with the capacity to capture HIV and HLA-II expression levels. *See* Figs. 1 and 3. On the other hand, maturation of iDCs with ITIP after (iDC+ITIP), rather than before (mDC ITIP), antigen uptake, did not improve HLA-II antigen presentation. *See* Fig. 2. These results highlight that DC maturation prior or after antigen loading does not guarantee efficient CD4⁺ T cell activation.

The type of activation (ITIP or LPS induced) also determines the capacity to process and present antigen. It has been reported that the presence of TLR4 ligands (such as LPS) together with the antigen cargo in phagosomes promotes antigen presentation to CD4⁺ T cells. In addition, DC maturation with LPS facilitates antigen proteolysis and efficient peptide-HLA-II complexes formation by lysosome acidification. *See* Manickasingham S, et al., J. Immunol. 2000; 165:5027-5034, Blander J, et al., Nature. 2006; 440:808-812 and Trombetta E, et al., Science 2003; 299:1400-1403. Interestingly, DCs loaded with integrase-deficient HIV_{NL4-3ΔIN} induced an activation comparable to DCs loaded with HIV_{NL4-3} in both HIV-specific CD8⁺ and CD4⁺ T cell clones, further demonstrating that no viral replication was needed for HIV-derived antigen presentation. HIV_{NL4-3ΔIN} represents a promising immunogen for the development of HIV vaccine since it is processed and presented by DC as wild type HIV.

## Claims

1. A method for obtaining an antigen-loaded antigen-presenting cell *in vitro* comprising contacting an immature dendritic cell with an immunogen comprising said antigen under conditions adequate for processing of the immunogen and presentation by the antigen-presenting cell.

2. A method according to claim 1 further comprising placing the immature dendritic cell under conditions adequate for maturation of said immature dendritic cell into a mature dendritic cell wherein the step of maturation of the immature dendritic cell is carried out simultaneously with the step of contacting the immature dendritic cell with an immunogen during at least part of said contacting step.

3. The method according to claim 2 wherein the conditions adequate for maturation of the immature dendritic cell into a mature dendritic cell comprise the incubation of said cell in a medium comprising a dendritic cell maturation agent selected from the group consisting of:
(i) a TLR4 ligand and
(ii) a pro-inflammatory cytokine cocktail.

4. The method according to claim 3 wherein the TLR4 ligand is lipopolysaccharide (LPS).

5. The method according to claim 3 wherein the pro-inflammatory cytokine cocktail comprises at least an agonist of the IL-1 receptor, a gp130 utilizing cytokine and a TNF superfamily member.

6. The method according to claim 5 wherein the pro-inflammatory cytokine cocktail comprises a mixture of IL-1β, IL-6 and TNF-α.

7. The method according to any of claims 4 or 5 wherein the pro-inflammatory cytokine cocktail further comprises a prostaglandin.

8. The method according to any of claims 1 to 7 wherein the immunogen is a HIV immunogen.

9. The method according to claim 8 wherein the HIV immunogen is a HIV particle.

10. The method according to claim 9 wherein the HIV-1 viral particle lacks a functional integrase protein.

11. An antigen-loaded antigen-presenting cell obtainable by a method according to any of claims 1 to 10.

12. An immunogenic composition or a vaccine comprising an antigen-presenting cell according to claim 11.

13. An antigen-presenting cell according to claim 11 for use in medicine.

14. An antigen-presenting cell according to claim 11 for use in the treatment or prevention of a disease which requires an immune response against the antigen which is loaded in the antigen-presenting cell.

15. An HIV-1 viral particle lacking a functional integrase protein that contains a deletion in the integrase protein or lacks the integrase protein.

16. A HIV-1 viral genome **characterized by** lacking partially or completely the integrase encoding region, a vector comprising a HIV-1 viral genome **characterized by** lacking partially or completely the integrase encoding region, or a cell comprising a HIV-1 viral genome **characterized by** lacking partially or completely the integrase encoding region or a vector comprising a HIV-1 viral genome **characterized by** lacking partially or completely the integrase encoding region for use in medicine.

17. A HIV-1 viral genome **characterized by** lacking partially or completely the integrase encoding region, a vector comprising a HIV-1 viral genome **characterized by** lacking partially or completely the integrase encoding region, or a cell comprising a HIV-1 viral genome **characterized by** lacking partially or completely the integrase encoding region or a vector comprising a HIV-1 viral genome **characterized by** lacking partially or completely the integrase encoding region for use in the treatment or prevention of a HIV infection or a disease associated with a HIV infection.

18. A method for obtaining a dendritic cell loaded with a HIV-1 immunogen *in vitro* comprising loading a dendritic cell with a HIV-1 viral particle lacking functional integrase protein.

19. A method according to claim 18 wherein the dendritic cell which is loaded with the HIV-1 viral particle has been by maturation of an immature dendritic cell using a TLR4 agonist.

20. A dendritic cell loaded with a HIV-1 viral particle lacking a functional integrase protein obtainable by a method according to any of claims 18 or 19.

21. An immunogenic composition or a vaccine comprising a dendritic cell according to claim 20.

22. A dendritic cell according to claim 20 for use in medicine.

23. A dendritic cell according to claim 20 for use in the treatment or prevention of a HIV infection or a disease associated with a HIV infection.


SEQUENCE LISTING

<110> LABORATORIOS DEL DR.ESTEVE, S.A.
FUNDACIÓ PRIVADA INSTITUT DE RECERCA DE LA SIDA-CAIXA
INSTITUCIÓ CATALANA DE RECERCA I ESTUDIS AVANÇATS

<120> HIV-1 INTEGRASE DEFICIENT IMMUNOGEN AND METHODS FOR LOADING
DENDRITIC CELLS WITH SAID IMMUNOGENS

<130> P7900EP00

<160> 3

<170> PatentIn version 3.5

<210> 1
<211> 13943
<212> DNA
<213> Artificial Sequence

<220>
<223> pNL4-3 delta integrase plasmid

<400> 1
tggaagggct aatttggtcc caaaaaagac aagagatcct tgatctgtgg atctaccaca 60

cacaaggcta cttccctgat tggcagaact acacaccagg gccagggatc agatatccac 120

tgacctttgg atggtgcttc aagttagtac cagttgaacc agagcaagta gaagaggcca 180

aataaggaga gaagaacagc ttgttacacc ctatgagcca gcatgggatg gaggacccgg 240

agggagaagt attagtgtgg aagtttgaca gcctcctagc atttcgtcac atggcccgag 300

agctgcatcc ggagtactac aaagactgct gacatcgagc tttctacaag ggactttccg 360

ctggggactt tccagggagg tgtggcctgg gcgggactgg ggagtggcga gccctcagat 420

gctacatata agcagctgct ttttgcctgt actgggtctc tctggttaga ccagatctga 480

gcctgggagc tctctggcta actagggaac ccactgctta agcctcaata aagcttgcct 540

tgagtgctca aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagtaaa gccagaggag atctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcggta ttaagcgggg gagaattaga taaatgggaa 840

aaaattcggt taaggccagg gggaaagaaa caatataaac taaaacatat agtatgggca 900

agcagggagc tagaacgatt cgcagttaat cctggccttt tagagacatc agaaggctgt 960

agacaaatac tgggacagct acaaccatcc cttcagacag gatcagaaga acttagatca 1020

ttatataata caatagcagt cctctattgt gtgcatcaaa ggatagatgt aaaagacacc 1080

aaggaagcct tagataagat agaggaagag caaaacaaaa gtaagaaaaa ggcacagcaa 1140

gcagcagctg acacaggaaa caacagccag gtcagccaaa attaccctat agtgcagaac 1200

ctccaggggc aaatggtaca tcaggccata tcacctagaa ctttaaatgc atgggtaaaa 1260

gtagtagaag agaaggcttt cagcccagaa gtaataccca tgttttcagc attatcagaa 1320

ggagccaccc cacaagattt aaataccatg ctaaacacag tggggggaca tcaagcagcc 1380

atgcaaatgt taaaagagac catcaatgag gaagctgcag aatgggatag attgcatcca 1440

gtgcatgcag ggcctattgc accaggccag atgagagaac caaggggaag tgacatagca 1500

ggaactacta gtacccttca ggaacaaata ggatggatga cacataatcc acctatccca 1560

gtaggagaaa tctataaaag atggataatc ctgggattaa ataaaatagt aagaatgtat 1620

agccctacca gcattctgga cataagacaa ggaccaaagg aaccctttag agactatgta 1680

gaccgattct ataaaactct aagagccgag caagcttcac aagaggtaaa aaattggatg 1740

acagaaacct tgttggtcca aaatgcgaac ccagattgta agactatttt aaaagcattg 1800

ggaccaggag cgacactaga agaaatgatg acagcatgtc agggagtggg gggacccggc 1860

cataaagcaa gagttttggc tgaagcaatg agccaagtaa caaatccagc taccataatg 1920

atacagaaag gcaattttag gaaccaaaga aagactgtta agtgtttcaa ttgtggcaaa 1980

gaagggcaca tagccaaaaa ttgcagggcc cctaggaaaa agggctgttg gaaatgtgga 2040

aaggaaggac accaaatgaa agattgtact gagagacagg ctaatttttt agggaagatc 2100

tggccttccc acaagggaag gccagggaat tttcttcaga gcagaccaga gccaacagcc 2160

ccaccagaag agagcttcag gtttggggaa gagacaacaa ctccctctca gaagcaggag 2220

ccgatagaca aggaactgta tcctttagct tccctcagat cactctttgg cagcgacccc 2280

tcgtcacaat aaagataggg gggcaattaa aggaagctct attagataca ggagcagatg 2340

atacagtatt agaagaaatg aatttgccag gaagatggaa accaaaaatg atagggggaa 2400

ttggaggttt tatcaaagta ggacagtatg atcagatact catagaaatc tgcggacata 2460

aagctatagg tacagtatta gtaggaccta cacctgtcaa cataattgga agaaatctgt 2520

tgactcagat tggctgcact ttaaattttc ccattagtcc tattgagact gtaccagtaa 2580

aattaaagcc aggaatggat ggcccaaaag ttaaacaatg gccattgaca gaagaaaaaa 2640

taaaagcatt agtagaaatt tgtacagaaa tggaaaagga aggaaaaatt tcaaaaattg 2700

ggcctgaaaa tccatacaat actccagtat ttgccataaa gaaaaaagac agtactaaat 2760

ggagaaaatt agtagatttc agagaactta ataagagaac tcaagatttc tgggaagttc 2820

aattaggaat accacatcct gcagggttaa aacagaaaaa atcagtaaca gtactggatg 2880

tgggcgatgc atatttttca gttcccttag ataaagactt caggaagtat actgcattta 2940

ccatacctag tataaacaat gagacaccag ggattagata tcagtacaat gtgcttccac 3000

agggatggaa aggatcacca gcaatattcc agtgtagcat gacaaaaatc ttagagcctt 3060

ttagaaaaca aaatccagac atagtcatct atcaatacat ggatgatttg tatgtaggat 3120

ctgacttaga aatagggcag catagaacaa aaatagagga actgagacaa catctgttga 3180

ggtggggatt taccacacca gacaaaaaac atcagaaaga acctccattc ctttggatgg 3240

gttatgaact ccatcctgat aaatggacag tacagcctat agtgctgcca gaaaaggaca 3300

gctggactgt caatgacata cagaaattag tgggaaaatt gaattgggca agtcagattt 3360

atgcagggat taaagtaagg caattatgta aacttcttag gggaaccaaa gcactaacag 3420

aagtagtacc actaacagaa gaagcagagc tagaactggc agaaaacagg gagattctaa 3480

aagaaccggt acatggagtg tattatgacc catcaaaaga cttaatagca gaaatacaga 3540

agcaggggca aggccaatgg acatatcaaa tttatcaaga gccatttaaa aatctgaaaa 3600

caggaaaata tgcaagaatg aagggtgccc acactaatga tgtgaaacaa ttaacagagg 3660

cagtacaaaa aatagccaca gaaagcatag taatatgggg aaagactcct aaatttaaat 3720

tacccataca aaaggaaaca tgggaagcat ggtggacaga gtattggcaa gccacctgga 3780

ttcctgagtg ggagtttgtc aatacccctc ccttagtgaa gttatggtat cagttagaga 3840

aagaacccat aataggagca gaaactttct atgtagatgg ggcagccaat agggaaacta 3900

aattaggaaa agcaggatat gtaactgaca gaggaagaca aaaagttgtc cccctaacgg 3960

acacaacaaa tcagaagact gagttacaag caattcatct agctttgcag gattcgggat 4020

tagaagtaaa catagtgaca gactcacaat atgcattggg aatcattcaa gcacaaccag 4080

ataagagtga atcagagtta gtcagtcaaa taatagagca gttaataaaa aaggaaaaag 4140

tctacctggc atgggtacct ctagaggatc ctctagacct gcaggcatat gtatatttca 4200

aggaaagcta aggactggtt ttatagacat cactatgaaa gtactaatcc aaaaataagt 4260

tcagaagtac acatcccact aggggatgct aaattagtaa taacaacata ttggggtctg 4320

catacaggag aaagagactg gcatttgggt cagggagtct ccatagaatg gaggaaaaag 4380

agatatagca cacaagtaga ccctgaccta gcagaccaac taattcatct gcactatttt 4440

gattgttttt cagaatctgc tataagaaat accatattag gacgtatagt tagtcctagg 4500

tgtgaatatc aagcaggaca taacaaggta ggatctctac agtacttggc actagcagca 4560

ttaataaaac caaaacagat aaagccacct ttgcctagtg ttaggaaact gacagaggac 4620

agatggaaca agccccagaa gaccaagggc cacagaggga gccatacaat gaatggacac 4680

tagagctttt agaggaactt aagagtgaag ctgttagaca ttttcctagg atatggctcc 4740

ataacttagg acaacatatc tatgaaactt acggggatac ttgggcagga gtggaagcca 4800

taataagaat tctgcaacaa ctgctgttta tccatttcag aattgggtgt cgacatagca 4860

gaataggcgt tactcgacag aggagagcaa gaaatggagc cagtagatcc tagactagag 4920

ccctggaagc atccaggaag tcagcctaaa actgcttgta ccaattgcta ttgtaaaaag 4980

tgttgctttc attgccaagt ttgtttcatg acaaaagcct taggcatctc ctatggcagg 5040

aagaagcgga gacagcgacg aagagctcat cagaacagtc agactcatca agcttctcta 5100

tcaaagcagt aagtagtaca tgtaatgcaa cctataatag tagcaatagt agcattagta 5160

gtagcaataa taatagcaat agttgtgtgg tccatagtaa tcatagaata taggaaaata 5220

ttaagacaaa gaaaaataga caggttaatt gatagactaa tagaaagagc agaagacagt 5280

ggcaatgaga gtgaaggaga agtatcagca cttgtggaga tgggggtgga aatggggcac 5340

catgctcctt gggatattga tgatctgtag tgctacagaa aaattgtggg tcacagtcta 5400

ttatggggta cctgtgtgga aggaagcaac caccactcta ttttgtgcat cagatgctaa 5460

agcatatgat acagaggtac ataatgtttg ggccacacat gcctgtgtac ccacagaccc 5520

caacccacaa gaagtagtat tggtaaatgt gacagaaaat tttaacatgt ggaaaaatga 5580

catggtagaa cagatgcatg aggatataat cagtttatgg gatcaaagcc taaagccatg 5640

tgtaaaatta accccactct gtgttagttt aaagtgcact gatttgaaga atgatactaa 5700

taccaatagt agtagcggga gaatgataat ggagaaagga gagataaaaa actgctcttt 5760

caatatcagc acaagcataa gagataaggt gcagaaagaa tatgcattct tttataaact 5820

tgatatagta ccaatagata ataccagcta taggttgata agttgtaaca cctcagtcat 5880

tacacaggcc tgtccaaagg tatcctttga gccaattccc atacattatt gtgccccggc 5940

tggttttgcg attctaaaat gtaataataa gacgttcaat ggaacaggac catgtacaaa 6000

tgtcagcaca gtacaatgta cacatggaat caggccagta gtatcaactc aactgctgtt 6060

aaatggcagt ctagcagaag aagatgtagt aattagatct gccaatttca cagacaatgc 6120

taaaaccata atagtacagc tgaacacatc tgtagaaatt aattgtacaa gacccaacaa 6180

caatacaaga aaaagtatcc gtatccagag gggaccaggg agagcatttg ttacaatagg 6240

aaaaatagga aatatgagac aagcacattg taacattagt agagcaaaat ggaatgccac 6300

tttaaaacag atagctagca aattaagaga acaatttgga aataataaaa caataatctt 6360

taagcaatcc tcaggagggg acccagaaat tgtaacgcac agttttaatt gtggagggga 6420

atttttctac tgtaattcaa cacaactgtt taatagtact tggtttaata gtacttggag 6480

tactgaaggg tcaaataaca ctgaaggaag tgacacaatc acactcccat gcagaataaa 6540

acaatttata aacatgtggc aggaagtagg aaaagcaatg tatgcccctc ccatcagtgg 6600

acaaattaga tgttcatcaa atattactgg gctgctatta acaagagatg gtggtaataa 6660

caacaatggg tccgagatct tcagacctgg aggaggcgat atgagggaca attggagaag 6720

tgaattatat aaatataaag tagtaaaaat tgaaccatta ggagtagcac ccaccaaggc 6780

aaagagaaga gtggtgcaga gagaaaaaag agcagtggga ataggagctt tgttccttgg 6840

gttcttggga gcagcaggaa gcactatggg ctgcacgtca atgacgctga cggtacaggc 6900

cagacaatta ttgtctgata tagtgcagca gcagaacaat ttgctgaggg ctattgaggc 6960

gcaacagcat ctgttgcaac tcacagtctg gggcatcaaa cagctccagg caagaatcct 7020

ggctgtggaa agatacctaa aggatcaaca gctcctgggg atttggggtt gctctggaaa 7080

actcatttgc accactgctg tgccttggaa tgctagttgg agtaataaat ctctggaaca 7140

gatttggaat aacatgacct ggatggagtg ggacagagaa attaacaatt acacaagctt 7200

aatacactcc ttaattgaag aatcgcaaaa ccagcaagaa aagaatgaac aagaattatt 7260

ggaattagat aaatgggcaa gtttgtggaa ttggtttaac ataacaaatt ggctgtggta 7320

tataaaatta ttcataatga tagtaggagg cttggtaggt ttaagaatag tttttgctgt 7380

actttctata gtgaatagag ttaggcaggg atattcacca ttatcgtttc agacccacct 7440

cccaatcccg aggggacccg acaggcccga aggaatagaa gaagaaggtg gagagagaga 7500

cagagacaga tccattcgat tagtgaacgg atccttagca cttatctggg acgatctgcg 7560

gagcctgtgc ctcttcagct accaccgctt gagagactta ctcttgattg taacgaggat 7620

tgtggaactt ctgggacgca gggggtggga agccctcaaa tattggtgga atctcctaca 7680

gtattggagt caggaactaa agaatagtgc tgttaacttg ctcaatgcca cagccatagc 7740

agtagctgag gggacagata gggttataga agtattacaa gcagcttata gagctattcg 7800

ccacatacct agaagaataa gacagggctt ggaaaggatt ttgctataag atgggtggca 7860

agtggtcaaa aagtagtgtg attggatggc ctgctgtaag ggaaagaatg agacgagctg 7920

agccagcagc agatggggtg ggagcagtat ctcgagacct agaaaaacat ggagcaatca 7980

caagtagcaa tacagcagct aacaatgctg cttgtgcctg gctagaagca caagaggagg 8040

aagaggtggg ttttccagtc acacctcagg tacctttaag accaatgact tacaaggcag 8100

ctgtagatct tagccacttt ttaaaagaaa aggggggact ggaagggcta attcactccc 8160

aaagaagaca agatatcctt gatctgtgga tctaccacac acaaggctac ttccctgatt 8220

ggcagaacta cacaccaggg ccaggggtca gatatccact gacctttgga tggtgctaca 8280

agctagtacc agttgagcca gataaggtag aagaggccaa taaaggagag aacaccagct 8340

tgttacaccc tgtgagcctg catggaatgg atgaccctga gagagaagtg ttagagtgga 8400

ggtttgacag ccgcctagca tttcatcacg tggcccgaga gctgcatccg gagtacttca 8460

agaactgctg acatcgagct tgctacaagg gactttccgc tggggacttt ccagggaggc 8520

gtggcctggg cgggactggg gagtggcgag ccctcagatg ctgcatataa gcagctgctt 8580

tttgcctgta ctgggtctct ctggttagac cagatctgag cctgggagct ctctggctaa 8640

ctagggaacc cactgcttaa gcctcaataa agcttgcctt gagtgcttca agtagtgtgt 8700

gcccgtctgt tgtgtgactc tggtaactag agatccctca gaccctttta gtcagtgtgg 8760

aaaatctcta gcacccccca ggaggtagag gttgcagtga gccaagatcg cgccactgca 8820

ttccagcctg ggcaagaaaa caagactgtc taaaataata ataataagtt aagggtatta 8880

aatatattta tacatggagg tcataaaaat atatatattt gggctgggcg cagtggctca 8940

cacctgcgcc cggccctttg ggaggccgag gcaggtggat cacctgagtt tgggagttcc 9000

agaccagcct gaccaacatg gagaaacccc ttctctgtgt atttttagta gattttattt 9060

tatgtgtatt ttattcacag gtatttctgg aaaactgaaa ctgtttttcc tctactctga 9120

taccacaaga atcatcagca cagaggaaga cttctgtgat caaatgtggt gggagaggga 9180

ggttttcacc agcacatgag cagtcagttc tgccgcagac tcggcgggtg tccttcggtt 9240

cagttccaac accgcctgcc tggagagagg tcagaccaca gggtgagggc tcagtcccca 9300

agacataaac acccaagaca taaacaccca acaggtccac cccgcctgct gcccaggcag 9360

agccgattca ccaagacggg aattaggata gagaaagagt aagtcacaca gagccggctg 9420

tgcgggagaa cggagttcta ttatgactca aatcagtctc cccaagcatt cggggatcag 9480

agtttttaag gataacttag tgtgtagggg gccagtgagt tggagatgaa agcgtaggga 9540

gtcgaaggtg tccttttgcg ccgagtcagt tcctgggtgg gggccacaag atcggatgag 9600

ccagtttatc aatccggggg tgccagctga tccatggagt gcagggtctg caaaatatct 9660

caagcactga ttgatcttag gttttacaat agtgatgtta ccccaggaac aatttgggga 9720

aggtcagaat cttgtagcct gtagctgcat gactcctaaa ccataatttc ttttttgttt 9780

tttttttttt atttttgaga cagggtctca ctctgtcacc taggctggag tgcagtggtg 9840

caatcacagc tcactgcagc ctcaacgtcg taagctcaag cgatcctccc acctcagcct 9900

gcctggtagc tgagactaca agcgacgccc cagttaattt ttgtattttt ggtagaggca 9960

gcgttttgcc gtgtggccct ggctggtctc gaactcctgg gctcaagtga tccagcctca 10020

gcctcccaaa gtgctgggac aaccggggcc agtcactgca cctggcccta aaccataatt 10080

tctaatcttt tggctaattt gttagtccta caaaggcagt ctagtcccca ggcaaaaagg 10140

gggtttgttt cgggaaaggg ctgttactgt ctttgtttca aactataaac taagttcctc 10200

ctaaacttag ttcggcctac acccaggaat gaacaaggag agcttggagg ttagaagcac 10260

gatggaattg gttaggtcag atctctttca ctgtctgagt tataattttg caatggtggt 10320

tcaaagactg cccgcttctg acaccagtcg ctgcattaat gaatcggcca acgcgcgggg 10380

agaggcggtt tgcgtattgg cgctcttccg cttcctcgct cactgactcg ctgcgctcgg 10440

tcgttcggct gcggcgagcg gtatcagctc actcaaaggc ggtaatacgg ttatccacag 10500

aatcagggga taacgcagga aagaacatgt gagcaaaagg ccagcaaaag gccaggaacc 10560

gtaaaaaggc cgcgttgctg gcgtttttcc ataggctccg cccccctgac gagcatcaca 10620

aaaatcgacg ctcaagtcag aggtggcgaa acccgacagg actataaaga taccaggcgt 10680

ttccccctgg aagctccctc gtgcgctctc ctgttccgac cctgccgctt accggatacc 10740

tgtccgcctt tctcccttcg ggaagcgtgg cgctttctca atgctcacgc tgtaggtatc 10800

tcagttcggt gtaggtcgtt cgctccaagc tgggctgtgt gcacgaaccc cccgttcagc 10860

ccgaccgctg cgccttatcc ggtaactatc gtcttgagtc caacccggta agacacgact 10920

tatcgccact ggcagcagcc actggtaaca ggattagcag agcgaggtat gtaggcggtg 10980

ctacagagtt cttgaagtgg tggcctaact acggctacac tagaaggaca gtatttggta 11040

tctgcgctct gctgaagcca gttaccttcg gaaaaagagt tggtagctct tgatccggca 11100

aacaaaccac cgctggtagc ggtggttttt ttgtttgcaa gcagcagatt acgcgcagaa 11160

aaaaaggatc tcaagaagat cctttgatct tttctacggg gtctgacgct cagtggaacg 11220

aaaactcacg ttaagggatt ttggtcatga gattatcaaa aaggatcttc acctagatcc 11280

ttttaaatta aaaatgaagt tttaaatcaa tctaaagtat atatgagtaa acttggtctg 11340

acagttacca atgcttaatc agtgaggcac ctatctcagc gatctgtcta tttcgttcat 11400

ccatagttgc ctgactcccc gtcgtgtaga taactacgat acgggagggc ttaccatctg 11460

gccccagtgc tgcaatgata ccgcgagacc cacgctcacc ggctccagat ttatcagcaa 11520

taaaccagcc agccggaagg gccgagcgca gaagtggtcc tgcaacttta tccgcctcca 11580

tccagtctat taattgttgc cgggaagcta gagtaagtag ttcgccagtt aatagtttgc 11640

gcaacgttgt tgccattgct acaggcatcg tggtgtcacg ctcgtcgttt ggtatggctt 11700

cattcagctc cggttcccaa cgatcaaggc gagttacatg atcccccatg ttgtgcaaaa 11760

aagcggttag ctccttcggt cctccgatcg ttgtcagaag taagttggcc gcagtgttat 11820

cactcatggt tatggcagca ctgcataatt ctcttactgt catgccatcc gtaagatgct 11880

tttctgtgac tggtgagtac tcaaccaagt cattctgaga atagtgtatg cggcgaccga 11940

gttgctcttg cccggcgtca atacgggata ataccgcgcc acatagcaga actttaaaag 12000

tgctcatcat tggaaaacgt tcttcggggc gaaaactctc aaggatctta ccgctgttga 12060

gatccagttc gatgtaaccc actcgtgcac ccaactgatc ttcagcatct tttactttca 12120

ccagcgtttc tgggtgagca aaaacaggaa ggcaaaatgc cgcaaaaaag ggaataaggg 12180

cgacacggaa atgttgaata ctcatactct tcctttttca atattattga agcatttatc 12240

agggttattg tctcatgagc ggatacatat ttgaatgtat ttagaaaaat aaacaaatag 12300

gggttccgcg cacatttccc cgaaaagtgc cacctgacgt ctaagaaacc attattatca 12360

tgacattaac ctataaaaat aggcgtatca cgaggccctt tcgtcttcaa gaactgcctc 12420

gcgcgtttcg gtgatgacgg tgaaaacctc tgacacatgc agctcccgga gacggtcaca 12480

gcttgtctgt aagcggatgc cgggagcaga caagcccgtc agggcgcgtc agcgggtgtt 12540

ggcgggtgtc ggggcgcagc catgacccag tcacgtagcg atagcggagt gtactggctt 12600

aactatgcgg catcagagca gattgtactg agagtgcacc atatgcggtg tgaaataccg 12660

cacagatgcg taaggagaaa ataccgcatc aggcgccatt cgccattcag gctgcgcaac 12720

tgttgggaag ggcgatcggt gcgggcctct tcgctattac gccaggggag gcagagattg 12780

cagtaagctg agatcgcagc actgcactcc agcctgggcg acagagtaag actctgtctc 12840

aaaaataaaa taaataaatc aatcagatat tccaatcttt tcctttattt atttatttat 12900

tttctatttt ggaaacacag tccttcctta ttccagaatt acacatatat tctatttttc 12960

tttatatgct ccagtttttt ttagaccttc acctgaaatg tgtgtataca aaatctaggc 13020

cagtccagca gagcctaaag gtaaaaaata aaataataaa aaataaataa aatctagctc 13080

actccttcac atcaaaatgg agatacagct gttagcatta aataccaaat aacccatctt 13140

gtcctcaata attttaagcg cctctctcca ccacatctaa ctcctgtcaa aggcatgtgc 13200

cccttccggg cgctctgctg tgctgccaac caactggcat gtggactctg cagggtccct 13260

aactgccaag ccccacagtg tgccctgagg ctgccccttc cttctagcgg ctgcccccac 13320

tcggctttgc tttccctagt ttcagttact tgcgttcagc caaggtctga aactaggtgc 13380

gcacagagcg gtaagactgc gagagaaaga gaccagcttt acagggggtt tatcacagtg 13440

caccctgaca gtcgtcagcc tcacaggggg tttatcacat tgcaccctga cagtcgtcag 13500

cctcacaggg ggtttatcac agtgcaccct tacaatcatt ccatttgatt cacaattttt 13560

ttagtctcta ctgtgcctaa cttgtaagtt aaatttgatc agaggtgtgt tcccagaggg 13620

gaaaacagta tatacagggt tcagtactat cgcatttcag gcctccacct gggtcttgga 13680

atgtgtcccc cgaggggtga tgactacctc agttggatct ccacaggtca cagtgacaca 13740

agataaccaa gacacctccc aaggctacca caatgggccg ccctccacgt gcacatggcc 13800

ggaggaactg ccatgtcgga ggtgcaagca cacctgcgca tcagagtcct tggtgtggag 13860

ggagggacca gcgcagcttc cagccatcca cctgatgaac agaacctagg gaaagcccca 13920

gttctactta caccaggaaa ggc 13943

<210> 2
<211> 9
<212> PRT
<213> Artificial Sequence

<220>
<223> optimal SL9 epitope

<400> 2

Ser Leu Tyr Asn Thr Val Ala Thr Leu
1               5

<210> 3
<211> 9
<212> PRT
<213> Artificial Sequence

<220>
<223> escape SL9 epitope

<400> 3

Ser Leu Phe Asn Thr Ile Ala Val Leu
1               5